# EUROPEAN PATENT SPECIFICATION

(11) **EP 0 550 635 B1**
(45) Date of publication and mention of the grant of the patent: **19.04.1995**
(21) Application number: 91918058.8
(22) Date of filing: 20.08.1991
(51) Int. Cl.: C07D 209/52, A61K 31/395, C07D 215/38, C07D 217/22, C07D 221/04, C07D 215/54, C07D 217/26

(54) **FUSED RING ANALOGS OF NITROGEN CONTAINING NONAROMATIC HETEROCYCLES**
RINGVERKNÜPFTE ANALOGE VON STICKSTOFFENTHALTENDEN NICHTAROMATISCHEN HETEROCYCLEN
ANALOGUES CYCLIQUES D'HETEROCYCLES NON AROMATIQUES CONTENANT DE L'AZOTE

(30) Priority: 28.09.1990 US 590423
(43) Date of publication of application: 14.07.1993
(73) Proprietor: PFIZER INC., Groton, CT 06340 (US)
(72) Inventor: DESAI, Manoj, C., Mystic, CT 06355 (US); HOWARD, Harry, R., Bristol, CT 06010 (US); ROSEN, Terry, J., East Lyme, CT 06333 (US)
(74) Representative: Moore, James William, Dr.
(86) International application number: US9105776
(87) International publication number: WO9206079

(56) References cited:
- EP-A- 0 050 881
- WO-A-90/05729
- FR-A- 1 555 552
- US-A- 3 452 026
- Chemical Abstracts, volume 86, no. 17, 25 April 1977, (Columbus, Ohio, US) see page 534, column 1, abstract 121189c, & JP, A, 76115484 (GRELAN PHARMACEUTICALCO., LTD) 12 October 1976

## Description

### Background of the Invention

The present invention relates to novel fused ring analogs of nitrogen containing heterocycles, pharmaceutical compositions comprising such compounds and the use of such compounds in the treatment and prevention of diseases mediated by or resulting, directly or indirectly, from an excess of substance P. The invention also relates to novel intermediates used in the synthesis of such compounds.

Substance P is a naturally occurring undecapeptide belonging to the tachykinin family of peptides, the latter being named because of their prompt stimulatory action on smooth muscle tissue. More specifically, substance P is a pharmacologically-active neuropeptide that is produced in mammals (having originally been isolated from gut) and possesses a characteristic amino acid sequence that is illustrated by D.F. Veber et al. in U.S. Patent No. 4,680,283. The wide involvement of substance P and other tachykinins in the pathophysiology of numerous diseases has been amply demonstrated in the art. For instance, substance P has recently been shown to be involved in the transmission of pain or migraine [see B.E.B. Sandberg et al., Journal of Medicinal Chemistry, Vol. 25, p. 1009 (1982)], as well as in central nervous system disorders such as anxiety and schizophrenia, in respiratory and inflammatory diseases such as asthma and rheumatoid arthritis, respectively, in rheumatic diseases such as fibrositis, and in gastrointestinal disorders and diseases of the GI tract such as ulcerative colitis and Crohn's disease, etc. (see D. Regoli in "Trends in Cluster Headache," edited by F. Sicuteri et al., Elsevier Scientific Publishers, Amsterdam, 1987, pp. 85-95).

PCT Patent Application PCT/US 90/00116, filed January 4, 1990, refers to carbotricyclic ring systems wherein one of the rings is substituted with an amino group and wherein one carbon atom in each of two of the rings may be replaced by a hetero atom, and states that they are useful as substance P antagonists. PCT Patent Application PCT/US 89/05338, filed November 20, 1989 and U.S. Patent Applications 07/557,442 and 07/532,525, filed July 23, 1990 and June 1, 1990, respectively, refer to amino substituted quinuclidine derivatives and state that they are useful as substance P antagonists.

### Summary of the Invention

The present invention relates to compounds of the formula 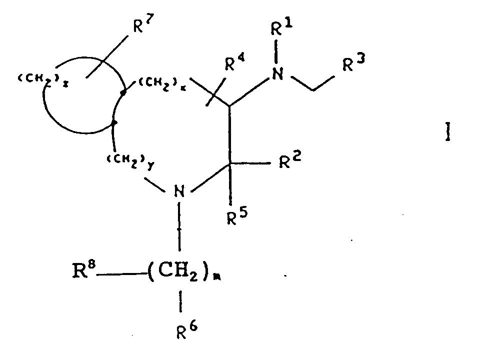
wherein x is an integer from zero to four;
y is an integer, from zero to four;
z is an integer from one to six;
the ring containing (CH₂)_{z} may contain from zero to three double bonds, and one of the carbons of (CH₂)_{z} may optionally be replaced by oxygen, sulfur or nitrogen;
m is an integer from zero to twelve, and any one of the carbon-carbon single bonds of (CH₂)ₘ may optionally be replaced by a carbon-carbon double or triple bond, and any one of the carbon atoms of (CH₂)ₘ may optionally be sustituted with R⁸ (as indicated by the attachment of R⁸ to (CH₂)m in structure I);
R¹ is hydrogen or (C₁-C₈) alkyl optionally substituted with hydroxy, alkoxy or fluoro;
R² is a radical selected from
aryl selected from phenyl and naphthyl; heteroaryl selected from indanyl, thienyl, furyl, pyridyl, thiazolyl, isothiazolyl, oxazolyl, isoxazolyl, triazolyl, tetrazolyl, and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl groups may optionally be substituted with one or more substituents independently selected from halo, nitro, (C₁-C₆) alkylamino, (C₁-C₆) alkoxy, trifluoromethyl, amino, (C₁-C₆)-alkylamino, 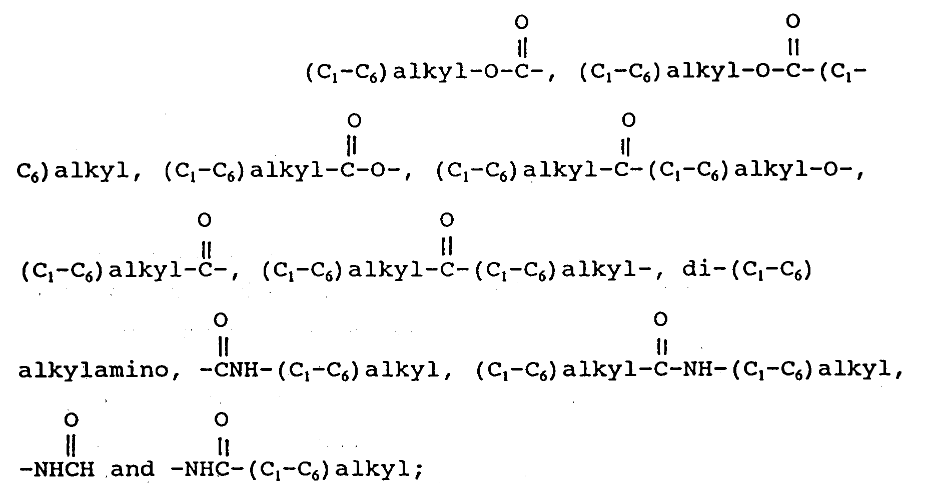
R⁵ is hydrogen or (C₁-C₆)alkyl;
or R² and R⁵, together with the carbon to which they are attached, form a saturated carbocyclic ring having from 3 to 7 carbon atoms, wherein one of said carbon atoms may optionally be replaced by oxygen, nitrogen or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl, and quinolyl; or cycloalkyl having from three to seven carbon atoms, wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, said substitutents being independently selected from halo, nitro, (C₁-C₆) alkyl, (C₁-C₆) alkoxy, trifluoromethyl, phenyl, amino, (C₁-C₆)alkylamino, 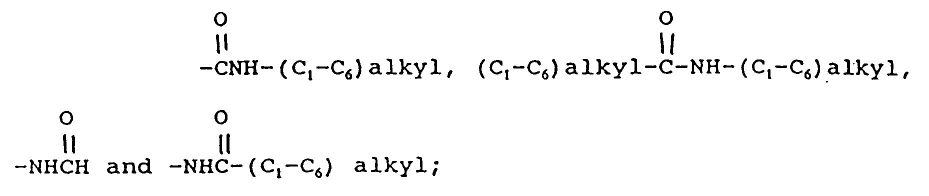
R⁴ may be attached to any atom of the nitrogen containing ring having an available bonding site and R⁷ may be attached to any atom of the (CH₂)_{z} containing ring having an available bonding site;
R⁴, R⁶, R⁷ and R⁸ are each independently selected from hydrogen, hydroxy, halo, amino, carboxy, carboxy(C₁-C₆)alkyl, (C₁-C₆)alkylamino, di-(C₁-C₆)alkylamino, (C₁-C₆)alkoxy, 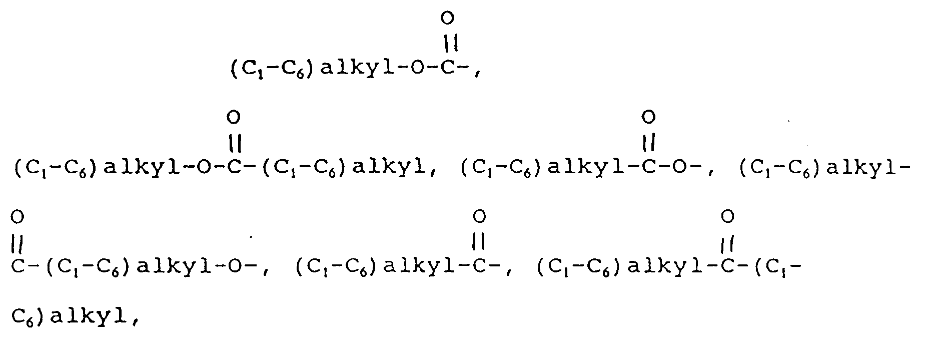
and the radicals set forth in the definition of R²
The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3-naphthoate)]salts.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "one or more substituents," as used herein, includes from one to the maximum number of substituents possible based on the number of available bonding sites.

The present invention also relates to compounds of the formula 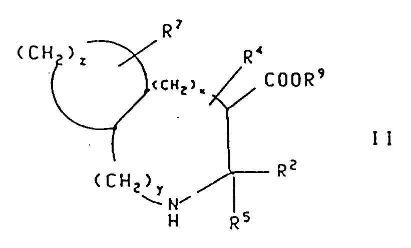
wherein R², R⁴, R⁵, R⁷, x, y and z are defined as for compounds of the formula I and R⁹ is hydrogen or (C₁-C₆)alkyl.

These compounds are useful as intermediates in the synthesis of compounds of the formula I.

The compounds of the formulae I and II have chiral centers and therefore exist in different enantiomeric forms. This invention relates to all optical isomers and all stereoisomers of compounds of the formula I, and mixtures thereof.

Formulae I and II above include compounds identical to those depicted but for the fact that one or more hydrogen or carbon atoms are replaced by radioactive isotopes thereof. Such radiolabelled compounds are useful as research and diagnostic tools in metabolism pharmokinetic studies and in binding assays. Specific applications in research include radioligand binding assays, autoradiography studies and in vivo binding studies, while specific applications in the diagnostic area include studies of the substance P receptor in the human brain in in vivo binding in the relevant tissues for inflammation, e.g. immune-type cells or cells that are directly involved in inflammatory bowel disorders and the like. Included among the radiolabelled forms of compounds of the formulae I and II are the tritium and C¹⁴ isotopes thereof.

Preferred compounds of the formula I are those wherein R¹, R⁴, R⁵, R⁶, R⁷ and R⁸ are hydrogen; R² is phenyl optionally substituted with one or more substituents independently selected from hydrogen, chlorine, fluorine, (C₁-C₆)alkoxy, (C₁-C₆)alkyl and trifluoromethyl; R³ is methoxyphenyl wherein the phenyl moiety is optionally substituted with one or more substitutents independently selected from hydrogen, chlorine, fluorine, (C₁-C₆)alkoxy, (C₁-C₆) alkyl or trifluoromethyl; m is zero; and each of x, y and z is an integer from zero to four.

Specific preferred compounds of the formula I are: [1α,3α,4β,5α]-4-(2-methoxybenzyl)amino-3-phenyl-2-azabicyclo[3.3.0]octane;
[1α,3α,4β,5α]-4-(2-chlorobenzyl)amino-3-phenyl-2-azabicyclo[3.3.0]octane;
[1α,3α,4α,5α]-4-(2-methoxybenzyl)amino-3-phenyl-2-azabicyclo[3.3.0]octane;
4-(2-methoxybenzyl)amino-3-phenyl-2-azabicyclo[4.4.0]decane; and
5-(2-methoxybenzyl)amino-4-benzhydryl-3-azabicyclo[4.1.0]heptane.
Other compounds of the formula I are:
10-(2-methoxybenzyl)amino-9-phenyl-8-azabicyclo[5.4.0]undecane;
4-(5-t-butyl-2-methoxybenzyl)amino-3-phenyl-2-azabicyclo[3.3.0]octane;
4-(5-isopropyl-2-methoxybenzyl)amino-3-phenyl-2-azabicyclo[3.3.0]octane;
4-(5-ethyl-2-methoxybenzyl)amino-3-phenyl-2-azabicyclo[3.3.0]octane;
4-(2-methoxy-5-n-propylbenzyl)amino-3-phenyl-2-azabicyclo[3.3.0]octane;
4-(5-t-butyl-2-isopropoxybenzyl)amino-3-phenyl-2-azabicyclo[3.3.0]octane;
4-(2-methoxy-5-phenylbenzyl)amino-3-phenyl-2-azabicyclo[3.3.0]octane;
4-(5-t-butyl-2-methoxybenzyl)amino-3-diphenylmethyl-2-azabicyclo[3.3.0]octane;
4-(5-t-butyl-2-methoxybenzyl)amino-3-phenyl-2-azabicyclo[4.4.0]decane;
4-(5-t-butyl-2-methoxybenzyl)amino-3-diphenylmethyl-2-azabicyclo[4.4.0]decane;
3-diphenylmethyl-4-(5-isopropyl-2-methoxybenzylamino-2-azabicyclo[4.1.0]heptane ;
9-(5-t-butyl-2-methoxybenzyl)amino-8-phenyl-7-azabicyclo[4.3.0]nonane;
10-(5-t-butyl-2-methoxybenzyl)amino-9-phenyl-8-azabicyclo[5.3.0]decane;
2-aza-4-(5-t-butyl-2-methoxybenzyl)amino-3-phenyl-7-oxabicyclo[3.3.0]octane;
2-aza-4-(5-ethyl-2-methoxybenzyl)amino-3-diphenylmethyl-7-oxabicyclo[3.3.0]octane;
2-aza-3-phenyl-4-(2-methoxybenzyl)amino-7-oxabicyclo[3.3.0]octane;
2-aza-3-diphenylmethyl-4-(2-methoxybenzyl)amino-7-oxabicyclo[3.3.0]octane;
2-aza-3-phenyl-4-(2-methoxybenzyl)amino-8-oxabicyclo[3.3.0]octane;
2-aza-3-diphenylmethyl-4-(2-methoxybenzyl)amino-8-oxabicyclo[3.3.0]octane;
5-(2-methoxybenzyl)amino-4-phenyl-3-azabicyclo[5.4.0]undecane;
2-(2-methoxybenzyl)amino-3-phenyl-4-azabicyclo[5.4.0]undecane;
5-(2-methoxylbenzyl)amino-4-phenyl-3-azabicyclo[5.3.0]decane;
2-(2-methoxybenzyl)amino-3-phenyl-4-azabicyclo[5.3.0]decane;
4-(2-methoxybenzyl)amino-3-phenyl-2-azabicyclo[4.3.0]nonane;
4-(2-methoxybenzyl)amino-3-phenyl-2-azabicyclo[5.3.0]decane;
4-(2-methoxybenzyl)amino-3-phenyl-2-azabicyclo[5.4.0]undecane;
4-(2-methoxybenzyl)amino-3-phenyl-2-azabicyclo[5.5.0]dodecane;
9-(2-methoxybenzyl)amino-8-phenyl-7-azabicyclo[4.3.0]nonane;
5-(2-methoxybenzyl)amino-4-phenyl-3-azabicyclo[5.3.0]decane;
5-(2-methoxybenzyl)amino-4-phenyl-3-azabicyclo[4.3.0]nonane;
5-(2-methoxybenzyl)amino-4-phenyl-3-azabicyclo[4.4.0]decane;
11-(2-methoxybenzyl)amino-10-phenyl-9-azabicyclo[5.4.0]undecane;
5-(2-methoxybenzyl)amino-4-phenyl-3-azabicyclo[5.1.0]octane;
4-(2-methoxylbenzyl)amino-3-phenyl-2-aza-8-oxabicyclo[4.3.0]nonane;
3-(diphenylmethyl)-4-(2-methoxybenzyl)amino-2-azabicyclo[3.3.0]octane;
10-(2,5-dimethoxybenzyl)amino-9-phenyl-8-azabicyclo[5.4.0]undecane;
5-(2,5-dimethoxybenzyl)amino-4-phenyl-3-azabicyclo[5.4.0]undecane;
2-(2,5-dimethoxybenzyl)amino-3-phenyl-4-azabicyclo[5.4.0]undecane;
5-(2,5-dimethoxylbenzyl)amino-4-phenyl-3-azabicyclo[5.3.0]decane;
2-(2,5-dimethoxybenzyl)amino-3-phenyl-4-azabicyclo[5.3.0]decane;
4-(2,5-dimethoxybenzyl)amino-3-phenyl-2-azabicyclo[4.3.0]nonane;
4-(2,5-dimethoxybenzyl)amino-3-phenyl-2-azabicyclo[5.3.0]decane;
4-(2,5-dimethoxybenzyl)amino-3-phenyl-2-azabicyclo[5.4.0]undecane;
4-(2,5-dimethoxybenzyl)amino-3-phenyl-2-azabicyclo[5.5.0]dodecane;
9-(2,5-dimethoxybenzyl)amino-8-phenyl-7-azabicyclo[4.3.0]nonane;
5-(2,5-dimethoxybenzyl)amino-4-phenyl-3-azabicyclo[5.3.0]decane;
5-(2,5-dimethoxybenzyl)amino-4-phenyl-3-azabicyclo[4.3.0]nonane;
5-(2,5-dimethoxybenzyl)amino-4-phenyl-3-azabicyclo[4.4.0]decane;
11-(2,5-dimethoxybenzyl)amino-10-phenyl-9-azabicyclo[5.4.0]undecane;
5-(2,5-dimethoxybenzyl)amino-4-phenyl-3-azabicyclo[5.1.0]octane;
4-(2,5-dimethoxylbenzyl)amino-3-phenyl-2-aza-8-oxabicyclo[4.3.0]nonane;
3-(diphenylmethyl)-4-(2,5-dimethoxybenzyl)amino-2-azabicyclo[3.3.0]octane;
10-(5-chloro-2-methoxybenzyl)amino-9-phenyl-8-azabicyclo[5.4.0]undecane;
5-(5-chloro-2-methoxybenzyl)amino-4-phenyl-3-azabicyclo[5.4.0]undecane;
2-(5-chloro-2-methoxybenzyl)amino-3-phenyl-4-azabicyclo[5.4.0]undecane;
5-(5-chloro-2-methoxylbenzyl)amino-4-phenyl-3-azabicyclo[5.3.0]undecane;
2-(5-chloro-2-methoxybenzyl)amino-3-phenyl-4-azabicyclo[5.3.0]undecane;
4-(5-chloro-2-methoxybenzyl)amino-3-phenyl-2-azabicyclo[4.3.0]nonane;
4-(5-chloro-2-methoxybenzyl)amino-3-phenyl-2-azabicyclo[5.3.0]decane;
4-(5-chloro-2-methoxybenzyl)amino-3-phenyl-2-azabicyclo[5.4.0]undecane;
4-(5-chloro-2-methoxybenzyl)amino-3-phenyl-2-azabicyclo[5.5.0]dodecane;
9-(5-chloro-2-methoxybenzyl)amino-8-phenyl-7-azabicyclo[4.3.0]nonane;
5-(5-chloro-2-methoxybenzyl)amino-4-phenyl-3-azabicyclo[5.3.0]decane;
5-(5-chloro-2-methoxybenzyl)amino-4-phenyl-3-azabicyclo[4.3.0]nonane;
5-(5-chloro-2-methoxybenzyl)amino-4-phenyl-3-azabicyclo[4.4.0]decane;
11-(5-chloro-2-methoxybenzyl)amino-10-phenyl-9-azabicyclo[5.4.0]undecane;
5-(5-chloro-2-methoxybenzyl)amino-4-phenyl-3-azabicyclo[5.1.0]octane;
4-(5-chloro-2-methoxylbenzyl)amino-3-phenyl-2-aza-8-oxabicyclo[4.3.0]nonane;
3-(diphenylmethyl)-4-(5-chloro-2-methoxybenzyl)amino-2-azabicyclo[3.3.0]octane;
10-(5-fluoro-2-methoxybenzyl)amino-9-phenyl-8-azabicyclo[5.4.0]undecane;
5-(5-fluoro-2-methoxybenzyl)amino-4-phenyl-3-azabicyclo[5.4.0]undecane;
2-(5-fluoro-2-methoxybenzyl)amino-3-phenyl-4-azabicyclo[5.4.0]undecane;
5-(5-fluoro-2-methoxylbenzyl)amino-4-phenyl-3-azabicyclo[5.3.0]undecane;
2-(5-fluoro-2-methoxybenzyl)amino-3-phenyl-4-azabicyclo[5.3.0]undecane;
4-(5-fluoro-2-methoxybenzyl)amino-3-phenyl-2-azabicyclo[4.3.0]nonane;
4-(5-fluoro-2-methoxybenzyl)amino-3-phenyl-2-azabicyclo[5.3.0]decane;
4-(5-fluoro-2-methoxybenzyl)amino-3-phenyl-2-azabicyclo[5.4.0]undecane;
4-(5-fluoro-2-methoxybenzyl)amino-3-phenyl-2-azabicyclo[5.5.0]dodecane;
9-(5-fluoro-2-methoxybenzyl)amino-8-phenyl-7-azabicyclo[4.3.0]nonane;
5-(5-fluoro-2-methoxybenzyl)amino-4-phenyl-3-azabicyclo[5.3.0]decane;
5-(5-fluoro-2-methoxybenzyl)amino-4-phenyl-3-azabicyclo[4.3.0]nonane;
5-(5-fluoro-2-methoxybenzyl)amino-4-phenyl-3-azabicyclo[4.4.0]decane;
11-(5-fluoro-2-methoxybenzyl)amino-10-phenyl-9-azabicyclo[5.4.0]undecane;
5-(5-fluoro-2-methoxybenzyl)amino-4-phenyl-3-azabicyclo[5.1.0]octane;
4-(5-fluoro-2-methoxylbenzyl)amino-3-phenyl-2-aza-8-oxabicyclo[4.3.0]nonane;
3-(diphenylmethyl)-4-(5-fluoro-2-methoxybenzyl)amino-2-azabicyclo[3.3.0]octane;
The compounds of the present invention are useful for treating or preventing a condition selected from the group consisting of inflammatory diseases (e.g., arthritis, psoriasis, asthma and inflammatory bowel disease), anxiety, depression or dysthymic disorders, colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis in a mammal, including a human.

The present invention also relates to a pharmaceutical composition comprising an amount of a compound of the formula I, or a pharmaceutically acceptable salt thereof, and a pharmaceutically acceptable carrier.

### Detailed Description of the Invention

The compounds of the formulae I and II may be prepared as described in the following reaction schemes and discussion. The formulae designated IA, IB, IC and ID represent different classes of compounds having the general formula I. Unless otherwise indicated, R¹, R², R³, R⁴, R⁵, R⁶, R⁷, R⁸, R⁹, x, y, z and m in the reaction schemes and discussion that follow are defined as above.

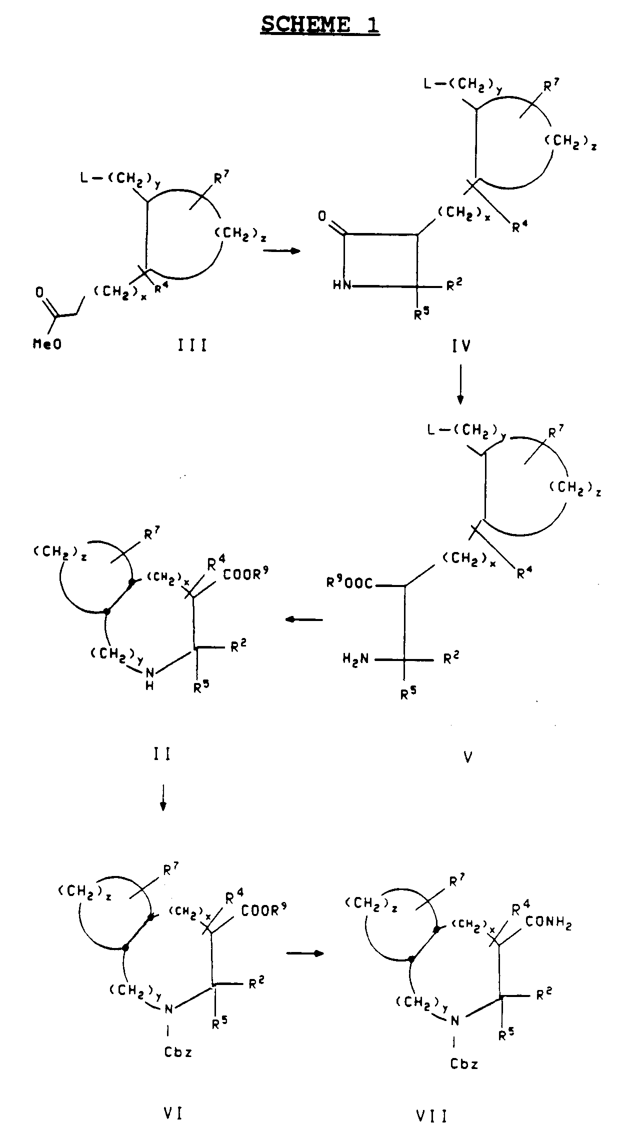

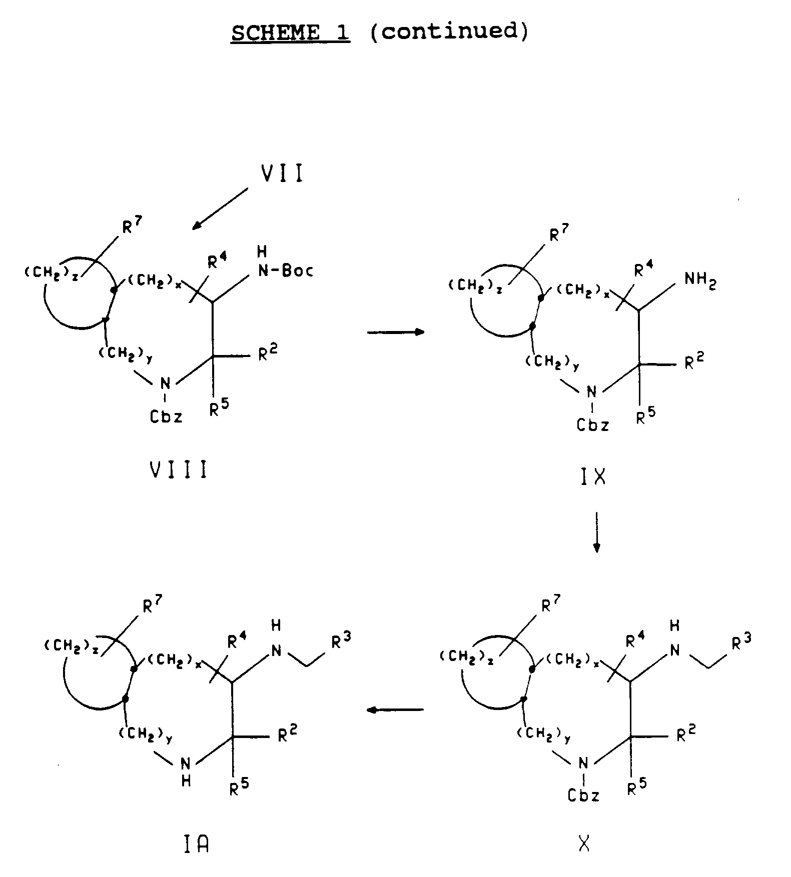

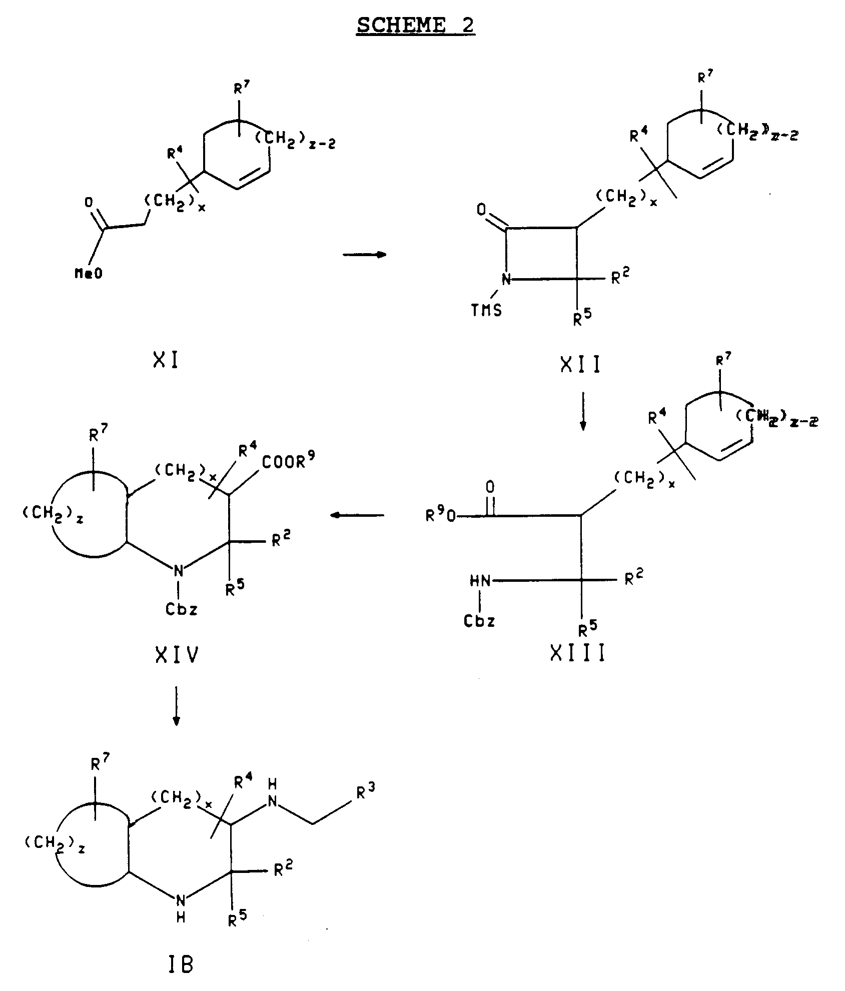

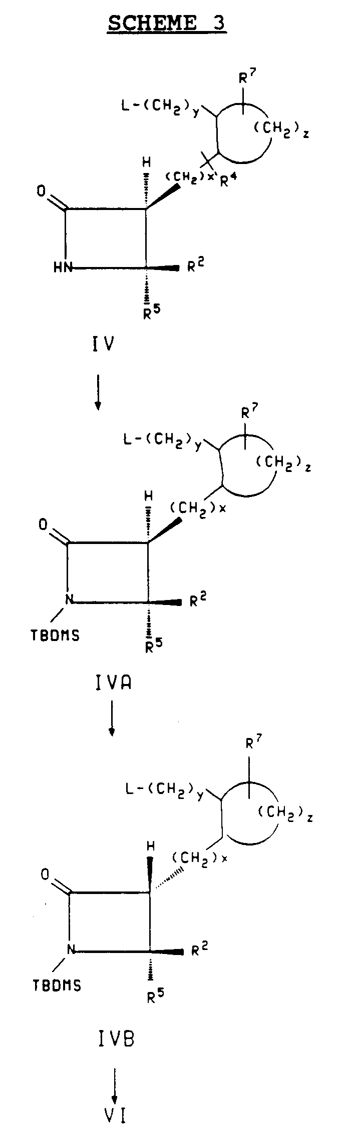

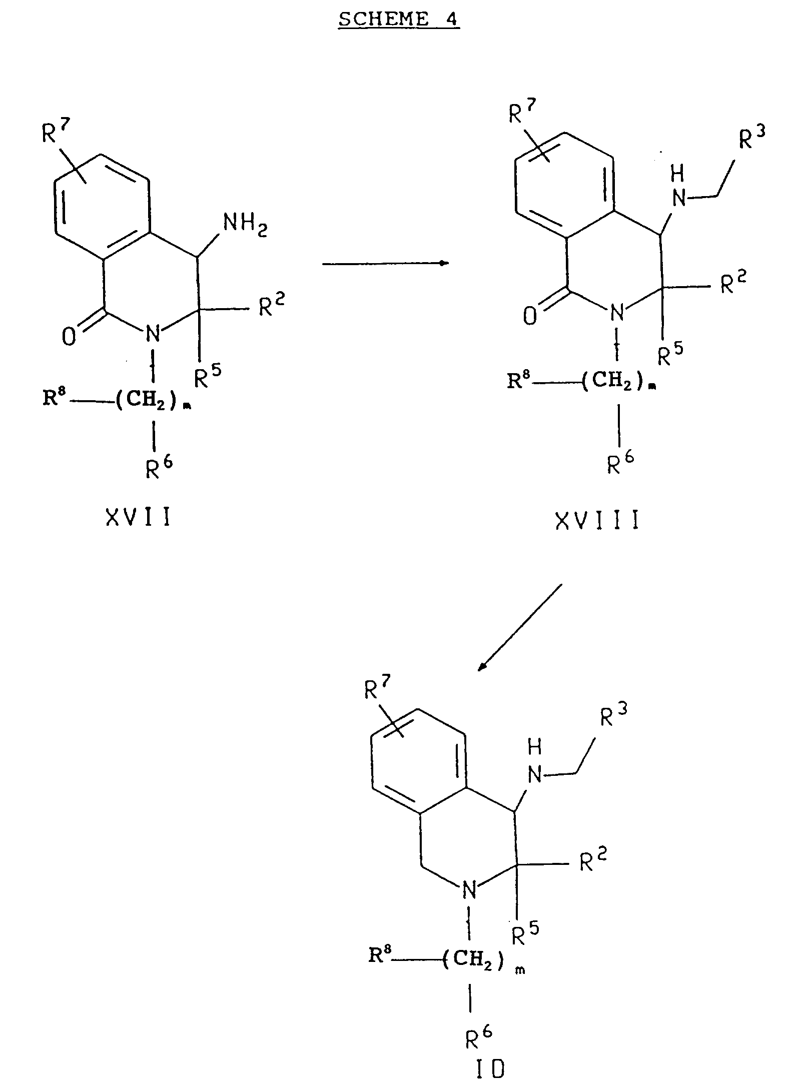

Scheme 1 illustrates the preparation of compounds of the formula IA. Formula IA represents compounds of the formula I wherein m is zero and both R¹ and R⁶ are hydrogen.

Referring to scheme 1, a compound of the formula III, wherein L is a leaving group, is reacted with N-(trimethylsilyl)benzaldimine. The reaction is generally carried out by adding the compound of formula III in tetrahydrofuran to lithium diisopropylamidemonotetrahydrofuran at a temperature from about -78°C to about -50°C, stirring the resulting mixture for about 0.25 to about 10 hours, and then adding N-(trimethylsilyl)benzaldimine to the mixture at approximately the same temperature over a period of about 5 minutes to about 1 hour. This reaction produces a β-lactam of the formula IV, wherein L is defined as it was for formula III. For example, L may be halo, hydroxy, carbomethoxy or a double bond (i.e.,

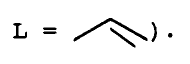

Cleavage of the β-lactam with an acid yields a compound of the formula V wherein R⁹ is (C₁-C₄)alkyl. Typically, this reaction is conducted using a concentrated acid such as concentrated sulfuric or perchloric acid, preferably sulfuric acid, in a polar solvent such as a lower alcohol, preferably methanol, for about 0.5 to about 16 hours. Suitable reaction temperatures range from about room temperature to about 150°C, with the reflux temperature of the solvent being preferred.

The cyclization of the compound of formula V to produce the corresponding compound having formula II is accomplished by heating the crude product of formula V from the foregoing reaction at a temperature from about 80 to about 140°C, preferably at about 100°C, for about 5 minutes to about 2 days, preferably for about 15 minutes, in a high boiling solvent such as dimethylformamide (DMF) or toluene, preferably DMF. This reaction is generally conducted in the presence of sodium iodide, tetrabutylammonium iodide or a similar salt, together with sodium bicarbonate, potassium carbonate or a similar salt. The compound of formula II is then treated with benzylchloroformate in a polar solvent such as water, water/acetone, chloroform, dichloroethane or ethyl acetate, in the presence of a base such as triethylamine or sodium bicarbonate, to yield the corresponding N-carbobenzyloxy compound of formula VI having the same stereochemistry with respect to the carbons to which R², R⁵ and COOR⁹ are attached. This reaction may be carried out at temperatures from about 0°C to about 100°C, preferably at about 25°C, for about 5 minutes to about 18 hours. Treatment of the compound of formula VI so formed with about five equivalents each of trimethyl aluminum and ammonium chloride in a nonpolar solvent such as benzene or toluene for about 0.5 to about 16 hours yields a compound having the formula VII and the same stereochemistry with respect to the carbons to which R², R⁵ and CONH₂ are attached. Reaction temperatures may range from about room temperature to about 100°C. About 50°C is preferred.

The conversion of the carboxamide group of the compound of formula VII to form a compound of the formula VIII having the same stereochemistry with respect to the carbons which R², R⁵ and NHBoc are attached (Boc = t-butoxycarbonyl), is generally accomplished by a Hoffmann degradation using lead tetraacetate in t-butanol. This reaction is typically carried out at a temperature from about room temperature to about the reflux temperature of the solvent, preferably at about the reflux temperature, for about 15 minutes to about 10 hours, preferably for about 3 to 5 hours. Alternatively, the desired compound of the formula VIII may be obtained via a Hoffman degradation using reagents such as bromine/sodium methoxide in methanol, iodobenzene bis(trifluoroacetate) in aqueous acetonitrile, sodium bromide, benzyltrimethyl ammonium tribromide or bis[trifluoroacetoxy]iodobenzene, under the foregoing reaction conditions, followed by treatment of the resulting amine with di-tert-butyl dicarbonate. Suitable solvents for the above alternative Hoffman degradations include inert aqueous solvents such as tetrahydrofuran (THF), dimethoxythane (DME) and dioxane. The reaction with di-tert-butyl dicarbonate is typically conducted in a reaction inert solvent such as dichloromethane or THF at a temperature from about 0°C to about 40°C, preferably at about room temperature.

Reaction of the compound of formula VIII with an acid yields a compound of the formula IX having the same stereochemistry with respect to the carbons to which R², R⁵ and NH₂ are attached. Examples of suitable acids are hydrochloric acid, trifluoroacetic acid and perchloric acid. The solvent is typically a polar solvent such as ethyl acetate, methylene chloride, dioxane, ethyl ether or THF, preferably ethyl acetate. This reaction is typically carried out at a temperature from about -10 to about 50°C, preferably at about 25°C, for about 0.5 to about 24 hours.

Reductive amination of a compound of the formula R³CHO in the presence of a compound of the formula IX from the above step yields a compound of the formula X having the same stereochemistry. Examples of reducing agents that may be used are hydrogen in the presence of a metal catalyst, sodium borohydride, sodium cyanoborohydride and sodium triacetoxyborohydride. This reaction is generally carried out in a polar solvent such as acetic acid or a lower alkanol, in the presence of a dehydrating agent such as molecular sieves, at a temperature from about 0 to about 50°C. Methanol is the preferred solvent and 25°C is the preferred temperature. It is also preferable that the pH of the reaction mixture be about 4 to about 5.

Alternatively, compounds of the formula X may be formed by acylating a compound of the formula IX with a compound having the formula R³COCl, and then reducing the resulting amide. The acylation is generally conducted in a polar solvent (e.g., dichloromethane, THF or ethyl ether), at a temperature from about 0 to about 60°C. The preferred solvent is dichloromethane and the preferred temperature is about 25°C. Examples of reducing agents that may be used to reduce the amide are lithium aluminum hydride and borane dimethyl sulfide. The reduction is typically carried out in a polar solvent (e.g., ether, THF or DME) at a temperature from about 0°C to about the reflux temperature of the solvent, preferably at about room temperature.

The compound of formula X is converted into a compound of the formula IA by reacting it with ammonium formate in the presence of palladium on charcoal (e.g., 10% palladium on charcoal). Usually, a polar solvent such as ethyl acetate or a lower alkanol is used, and the reaction is run at a temperature from about room temperature to about 150°C for about 0.5 to about 24 hours. Preferably, the reaction is conducted in ethanol at room temperature for about 3 to about 24 hours.

The compounds of formula IA prepared by the foregoing procedure may be converted into compounds that are identical but for the fact that R¹ is other than hydrogen as described below. A compound of the formula X is reacted with a compound of the formula R¹X wherein X is a leaving group such as halo (e.g. bromine, chlorine, fluorine, or iodine). This reaction is generally conducted in the presence of a base such as triethylamine or potassium t-butoxide in a polar solvent such as methylene chloride or dichloroethane, at a temperature from about room temperature to about 150°C. Preferably, the reaction is carried out at about the reflux temperature in methylene chloride in the presence of triethylamine. The product of this reaction may be converted to a compound having the formula IA by treatment with ammonium formate in the presence of palladium on charcoal, as described above.

Compounds of the formula IA may be converted into compounds of the formula I that are identical to them but for the fact that R⁶ is other than hydrogen as described below. A compound of the formula IA is reacted with a compound of the formula R⁶-(CH₂)ₘ-X, wherein X is a leaving group such as halo, wherein one of the carbon-carbon single bonds of said (CH₂)ₘ may optionally be replaced by a carbon-carbon double bond, and wherein one of the carbons of said (CH₂)ₘ may optionally be substituted with R⁸. This reaction is typically carried out in the presence of a base such as triethylamine or potassium t-butoxide, in a polar solvent such as methylene chloride or dichloroethane, at a temperature from about room temperature to about 150°C. Preferably, the reaction is carried out at the reflux temperature in methylene chloride in the presence of triethylamine.

Scheme 2 illustrates an alternate and preferred method of synthesizing compounds of the formula I wherein y is zero, z is greater than one and R⁵ is hydrogen. These compounds are represented in scheme 2 by formula IB.

Referring to scheme 2, a compound of the formula XI is reacted with a compound having the formula 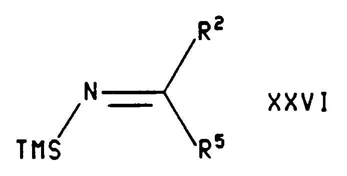
wherein R⁵ is hydrogen. This reaction is generally carried out by adding the compound of formula XI in tetrahydrofuran to lithium diisopropylamide monotetrahydrofuran at a temperature from about -78°C to about -50°C, stirring the resulting mixture for about 0.25 to about 10 hours, and then adding the compound of formula XXVI to the mixture at approximately the same temperature over a period of about 5 minutes to about 1 hour. This reaction produces a β-lactam of the formula XII.

Cleavage of the β-lactam with an acid followed by treatment with benzylchloroformate yields a compound of the formula XIII having the same stereochemisty, wherein R⁹ is (C₁-C₄)alkyl. Cleavage of the β-lactam is typically conducted using a concentrated acid such as concentrated sulfuric or perchloric acid, perferably sulfuric acid, in a polar solvent such as a lower alcohol, preferably methanol, for about 0.5 to about 16 hours. Suitable reaction temperatures range from about room temperature to about 150°C, with the reflux temperature of the solvent being preferred. The product of this reaction is a compound having a structure similar to that of formula XIII, except that the N-carbobenzyloxy group is replaced by hydrogen. Treatment of this compound with benzylchloroformate to produce a compound of the formula XIII having the same stereochemistry is generally carried out in a polar solvent such as water, water/acetone, chloroform, dichloroethane or ethyl acetate in the presence of a base. Examples of bases that may be used are triethylamine, sodium bicarbonate and potassium bicarbonate. This reaction may be conducted at temperatures from about 0°C to about 100°C, for a period of about 5 minutes to about 18 hours. Preferably, it is conducted at about 25°C.

The compound of formula XIII may be cyclized to form the corresponding compound having the formula XIV and the same stereochemistry with respect to the carbons to which R², R⁵ and COOR⁹ are attached, by first reacting it with mercuric trifluoroacetate, mercuric chloride or mercuric acetate, and then reacting the product of such reaction with sodium borohydride, lithium triacetoxyborohydride or a similar reducing agent. The reaction with the mercury salt is usually conducted in a polar solvent at a temperature from about -78°C to about 25°C. Suitable solvents include THF, acetonitrile and nitromethane. The reaction is carried out preferably using mercuric trifluoroacetate in THF at about 25°C. The reaction with sodium borohydride is generally carried out by adding an aqueous solution of sodium borohydride to the reaction mixture from the foregoing reaction at a temperature from about -78°C to about 0°C, preferably at about 0°C.

Compounds of the formula XIV may be converted to the corresponding compounds having the formula IB and the same stereochemistry with respect to the carbons to which R², R⁵ and NHR³ are attached by the method illustrated in scheme 1 and described above for converting compounds of the formula VI into compounds of the formula IA.

Compounds of the formula I may also be prepared using the procedure described in Example 8.

Scheme 3 illustrates a variation of scheme 1 that may be used to obtain compounds of the formula IA wherein R⁵ is hydrogen having a particular stereochemistry. Referring to scheme 3, the β-lactam of formula IV, which is produced in the first reaction of scheme 1, is reacted with t-butyldimethylsilyl (TBDMS) triflate . The reaction is typically conducted in a polar, reaction inert solvent in the presence of a hindered tertiary amine such as diisopropylethyl amine at a temperature from about -78°C to about 50°C. It is preferably conducted at about 0°C. Suitable solvents include methylene chloride, ether, THF and ethyl acetate, with methylene chloride being preferred. The product of this reaction is a compound of the formula IVA. This compound may be converted to the corresponding compound having the formula IVB by reacting it first with lithium diisopropylamide and then with a weak organic or inorganic acid. Generally, the reaction with lithium diisopropylamide is carried out in a reaction inert solvent such as THF, ether or DME, preferably THF, at a temperature from about -78°C to about 0°C, preferably at about -78°C. The reaction with a weak acid is generally accomplished by quenching the mixture from the above reaction with acetic acid, ethanol/methanol, water, aqueous ammonium chloride or trifluoroacetic acid, preferably acetic acid, in a reaction inert solvent such as THF, ether or hexane, preferably THF, at a temperature from about -78°C to about 0°C. A temperature of about 25°C is preferred.

The compounds of the formula IVB produced by the procedure of scheme 3 will have a stereochemistry opposite to that of the corresponding compounds of the formula IVA from which they were made, with respect to the carbon to which R² and R⁵ are attached and the carbon adjacent to it. These compounds of formula IVB may then be converted to the corresponding compounds of the formula IA by the procedure illustrated in scheme 1 and described above for converting compounds of the formula IV to compounds of the formula IA. By using the procedure of scheme 3 in combination with that of scheme 1, as described above, compounds of the formula IA having the cis configuration may be formed from the corresponding compounds of the formula IV having the cis configuration.

Similarly, the procedure of scheme 3 may be used in combination with the procedure of scheme 2 to produce compounds of the formula IB having a particular stereochemistry. Thus, compounds of the formula XII, as shown in scheme 2, having the cis configuration may be converted to the corresponding compounds of the formula 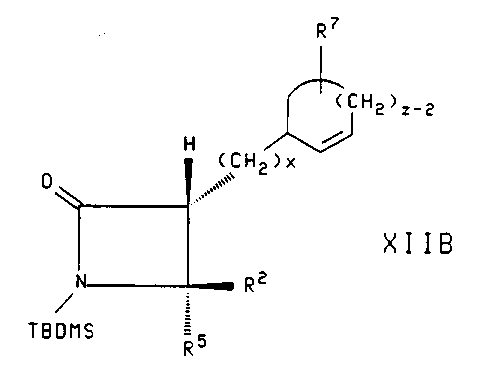
wherein R⁵ is hydrogen by a method analogous to the procedure described above for forming compounds of the formula IVB from compounds of the formula IV. The compounds of formula XIIB so formed may then be converted into the corresponding compounds of the formula IB having the same stereochemistry using the procedure illustrated in scheme 2 and described above for forming compounds of the formula IB from compounds of the formula XII.

An alternate and preferred method of synthesizing compounds of the formula I wherein m and y are zero, x is one, z is four, R¹ and R⁶ are both hydrogen, and the (CH₂)_{z} containing ring is saturated (hereinafter referred to as compounds of the formula IC) involves reducing a compound of the formula 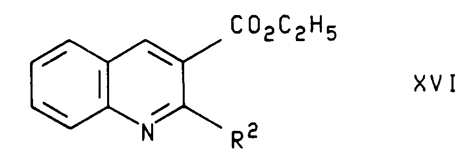
using hydrogen in the presence of an acid and a metal catalyst, and then converting the derived compound to a compound of the formula 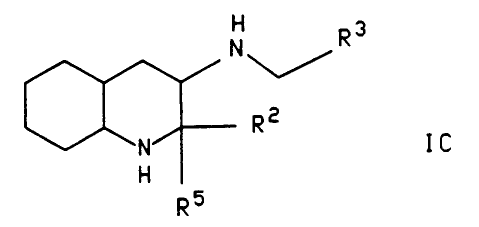
using the series of reactions illustrated in scheme 1 and described above for effecting the following transformations: II→VI→VII→IX→X→IA. The reduction of the compound having formula XVI is usually conducted using platinum oxide, platinum on carbon or palladium on carbon as the catalyst and acetic acid or hydrochloric acid/alcohol as the acid. Preferably, platinum oxide and acetic acid are used.

Scheme 4 depicts an alternate and preferred method of synthesizing compounds of the formula I wherein R¹ is hydrogen, z is 4, y is 1 and x is 0. These compounds are represented by formula ID in scheme 4.

Referring to scheme 4, a compound of XVII is converted into the corresponding compound having formula XVIII by a reductive amination using the procedure described above for forming compounds of the formula X from compounds of the formula IX.

Reduction of either the cis or trans isomer of the compound of formula XVIII, or a mixture thereof, yields a compound of the formula ID having the same stereochemistry. Reducing agents such as lithium aluminum hydride, borane in THF and sodium borohydride-titanium (IV) chloride are suitable; however, best results are obtained by using borane dimethylsulfide in THF. The reaction may be carried out at temperatures from about room temperature to about 150°C. It is preferably carried out at the reflux temperature of the solvent.

Compounds identical to those of the formulae IB, IC and ID but for the fact that R¹ or R⁶ is other than hydrogen may be prepared from the corresponding compounds of the formulae IB, IC and ID by the methods described above for synthesizing compounds identical to those of formula IA but for the fact that R¹ or R⁶ is other than hydrogen.

The preparation of other compounds of the formula I not specifically described above can be accomplished using combinations of the above reactions that will be clear to those skilled in the art in view of the foregoing disclosure.

In each of the reactions discussed or illustrated in schemes 1 to 4 above, pressure is not critical unless otherwise indicated. Pressures from about 0.5 atmospheres to about 5 atmospheres are generally acceptable, and ambient pressure, i.e. about 1 atmosphere, is preferred as a matter of convenience.

The compounds of the formula I and the pharmaceutically acceptable salts thereof (hereinafter referred to as the active compounds) are useful as substance P antagonists, i.e., they possess the ability to antagonize the effects of substance P at its receptor site in mammals, and therefore are able to function as therapeutic agents in the treatment of the aforementioned disorders and diseases in an afflicted mammal.

The compounds of the formula I which are basic in nature are capable of forming a wide variety of different salts with various inorganic and organic acids. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

The compounds of formula I and their pharmaceutically acceptable salts exhibit substance P receptor-binding activity and therefore are of value in the prevention and treatment of a wide variety of clinical conditions the treatment or prevention of which is effected or facilitated by a decrease in substance P mediated neurotransmission. Such conditions include inflammatory diseases (e.g., arthritis, psoriasis, asthma and inflammatory bowel disease), anxiety, depression or dysthymic disorders, colitis, psychosis, pain, allergies such as eczema and rhinitis, chronic obstructive airways disease, hypersensitivity disorders such as poison ivy, vasospastic diseases such as angina, migraine and Reynaud's disease, fibrosing and collagen diseases such as scleroderma and eosinophilic fascioliasis, reflex sympathetic dystrophy such as shoulder/hand syndrome, addiction disorders such as alcoholism, stress related somatic disorders, peripheral neuropathy, neuralgia, neuropathological disorders such as Alzheimer's disease, AIDS related dementia, diabetic neuropathy and multiple sclerosis, disorders related to immune enhancement or suppression such as systemic lupus erythematosus, and rheumatic diseases such as fibrositis. Hence, these compounds are readily adapted to therapeutic use as substance P antagonists for the control and/or treatment of any of the aforesaid clinical conditions in mammals, including humans.

The compounds of the formula I and the pharmaceutically acceptable salts thereof can be administered via the oral, parenteral or topical routes. In general, these compounds are most desirably administered in dosages ranging from about 5.0 mg up to about 1500 mg per day, although variations will necessarily occur depending upon the weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate while in other cases still larger doses may be employed without causing any harmful side effect provided that such higher dose levels are first divided into several small doses for administration throughout the day.

The active compounds of this invention may be administered alone or in combination with pharmaceutically acceptable carriers or diluents by either of the three routes previously indicated, and such administration can be carried out in single or multiple doses. More particularly, the active compounds of this invention can be administered in a wide variety of different dosage forms, i.e., they may be combined with various pharmaceutically acceptable inert carriers in the form of tablets, capsules, lozenges, troches, hard candies, powders, sprays, aerosols, creams, salves, suppositories, jellies, gels, pastes, lotions, ointments, aqueous suspensions, injectable solutions, elixirs, syrups, and the like. Such carriers include solids diluents or fillers, sterile aqueous media and various non-toxic organic solvents, etc. Moreover, oral pharmaceutical compositions can be suitably sweetened and/or flavored. In general, the active compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if desired, emulsifying and/or suspending agents as well, together with such diluents as water, ethanol, propylene glycol, glycerin and various combinations thereof.

For parenteral administration, solutions of a therapeutic compound of the present invention in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarteriolar, intramuscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin. This may be done, preferably, by way of creams, jellies, gels, pastes, ointments and the like, in accordance with standard pharmaceutical practice.

The activity of the compounds of the present invention as substance P antagonists may be determined by their ability to inhibit the binding of substance P at its receptor sites in bovine caudate or other mammalian tissue, employing radioactive ligands to visualize the tachykinin receptors by means of autoradiography. The substance P antagonist activity of the herein described compounds may be evaluated using the standard assay procedure described by M.A. Cascieri et al., as reported in the Journal of Biological Chemistry, Vol. 258, p. 5158 (1983) or variations thereof. This method essentially involves determining the concentration of the individual compound required to reduce by 50% the amount of radiolabelled substance P ligands at their receptor sites in said isolated cow or other mammalian tissues, thereby affording characteristic IC₅₀ values for each compound tested.

In one procedure, bovine caudate tissue is removed from a -70°C freezer and homogenized in 50 volumes (w./v.) of ice cold 50 Mm Tris (i.e., 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty minute period. The pellet is then resuspended in 40 volumes of ice-cold 50 Mm Tris buffer (pH 7.7) containing 2 mM of calcium chloride, 2 mM of magnesium chloride, 40 g/ml of bacitracin, 4»g/ml of leupeptin, 2»g of chymostatin and 200 g/ml of bovine serum albumin. This step completes the production of the tissue preparation.

The radioligand binding procedure is then carried out by initiating the reaction via the addition of 100 »l of the test compound made up to a concentration of 1»M, followed by the addition of 100 »l of radioactive ligand made up to a final concentration 0.5 mM and then finally by the addition of 800 »l of the tissue preparation produced as described above. The final volume is 1.0 ml. The reaction mixture is then vortexed and incubated at room temperature (ca. 20°C) for a period of 20 minutes. The tubes are then filtered using a cell harvester, and the glass fiber filters (Whatman GF/B) are washed four times with 50 mM Tris buffer (pH 7.7), the filters having previously been presoaked for a period of two hours prior to the filtering procedure. Radioactivity is then determined in a Beta counter at 53% counting efficiency, and the IC₅₀ values calculated using standard statistical methods.

The antipsychotic activity of the compounds of the present invention as neuroleptic agents for the control of various psychotic disorders is determined primarily by a study of their ability to suppress substance P-induced or substance P agonist induced hypermotility in guinea pigs. This study is carried out by first dosing the guinea pigs with a control compound or with an appropriate test compound of the present invention, then injecting the guinea pigs with substance P or a substance P agonist by intracerebral administration via canula and thereafter measuring their individual locomotor response to said stimulus.

The present invention is illustrated by the following examples. However, the invention is not limited to the specific details of these examples.

### EXAMPLE 1

### [1α,3α,4α,5α]-4-(2-Methoxybenzyl)amino-3-phenyl-2-azabicyclo[3.3.0]octane

### A. Cis-3-(2-cyclopentenyl)-4-phenyl-1-azetidin-2-one

To a cooled solution of lithium bis(trimethylsilyl)amide (1.0M in tetrahydrofuran, 200 mL, 200 mmoles) at -78°C was added benzaldehyde (19.7 gms, 189 mmoles). The reaction mixture was warmed to 0°C for 30 mins and recooled to -78°C. In another flask lithium diisopropylamide-monotetrahydrofuran (1.5M in cyclohexanes, 114 mL, 171 mmole) in tetrahydrofuran (150 ml) was cooled to -78°C and to it a solution of 2-cyclopentene-1-acetic acid, methyl ester (20 g, 142.8 mmole) in tetrahydrofuran (150 mL) was added slowly. The reaction mixture was stirred for 30 minutes at -78°C followed by the addition of the contents of the first flask via cannula over a 15 minute period. The cold bath was removed and the mixture was allowed to warm to room temperature (1.5 hours.) The resulting solution was diluted with 100 mL 1N HCl and stirred for 1 hour. The resulting solution was extracted with ether (3 x 350 mL). The organic phases were combined and washed with water, dried (anhydrous MgSO₄) and concentrated in vacuo to afford a residue which on trituration with ether-pentane afforded the desired β-lactam as white solid (20.6 gms). The mother liquor was concentrated and chromatographed. Elution with 30% ethyl acetate in hexane afforded additional cis-3-(2-cyclopentenyl)-4-phenyl-1-azetidin-2-one (4.14 gms, total yield 81%).

¹H NMR δ (CDCl₃) 1.03-1.15 (1H, m), 1.16-1.3 (1H, m), 1.8-2.3 (2H, m), 2.55-2.75 (1H, m), 3.23-3.48 (1H, m), 4.88 (1H, d, J=5.3 Hz), 4.95-5.05 (0.33H, m), 5.55-5.63 (0.33H, m), 5.64-5.7 (0.66H, m), 5.85-5.92 (0.66H, m), 6.1 (1H, bd), 7.2-7.4 (5H, m).

### B. Cis-3-(2-cyclopentenyl)-4-phenyl-1-(tert.-butyldimethylsilyl)azetidin-2-one

To a stirred solution of cis-3-(2-cyclopentenyl)-4-phenyl-1-azetidin-2-one (24.73 g, 116 mmoles), and diisopropylethyl amine (26.3 mL, 150 mmoles) in methylene chloride (400 mL) at 0°C, was added tert. butyldimethylsilyl triflate (31.5 mL, 139 mmoles). The reaction mixture was stirred for 15 minutes, and was then diluted with methylene chloride (200 ml) and with water. The organic phase was successively washed with 1N HCl (1 x 100 ml), 1N NaHCO₃ (1 x 100 ml) and water (2 x 100 mL). The methylene chloride layer was dried (anhydrous MgSO₄), filtered and concentrated under vacuum to afford pure cis-3-(2-cyclopentenyl)-4-phenyl-1-(tert.- butyldimethylsilyl)azetidin-2-one (33.76 gm, 89%).

¹H NMR δ (CDCl₃) 0.68 (3H, s), 0.95 (9H, s), 0.95-1.15 (2H, m), 1.8-2.15 (2H, m), 2.53-2.72) (1H, m), 3.3-3.4 (1H, m), 4.7 (1H, d, J=4Hz), 4.73-4.8 (0.33H, m), 5.5-5.57 (0.33H, m), 5.6-5.7 (0.66H, m), 5.9-6.0 (0.66H, m), 7.3-7.45 (5H, m).

### C. Trans-3-(2-cyclopentenyl)-4-phenyl-1-(tert.-butyldimethylsilyl)azetidin-2-one

To a stirred solution of cis-3-(2-cyclopentenyl)-4-phenyl-1-(tert-butyldimethylsilyl)azetidin-2-one (15.00 gms, 46 mmoles) in tetrahydrofuran (200 mL) at -78°C, was added lithium diisopropylamide in tetrahydrofuran (1.5M, 33.6 mL, 50 mmoles). The reaction mixture was then warmed to -50°C and stirred for 20 minutes. At the end of this period the reaction mixture was quenched with acetic acid (13.7 gms, 228 mmoles) in tetrahydrofuran (50 mL) and was warmed to room temperature (25°C). Thereafter, it was concentrated under vacuum, diluted with 1N HCl and extracted with ethyl ether (3 x 150 ml). The combined organic layers were washed with water (2 x 100 mL), dried (anhydrous MgSO₄) and filtered. The ethyl ether was removed under vacuum to afford an orange colored oil (17.5 g) which was loaded on flash silica gel column. Elution with 10% ethyl acetate in hexane afforded pure trans-3-(2-cyclopentenyl)-4-phenyl-1-(tert-butyldimethylsilyl)azetidin-2-one (6.86 gms, 46%).

¹H NMR (CDCl₃) δ 0.19 (3H, s), 0.91 (9H, s), 1.5-1.65 (1H, m), 2.0-2.15 (1H, m), 2.3-2.5 (2H, m), 3.1-3.25 (2H, m), 4.19 (1H, d, J=2.4 Hz), 5.65-5.75 (1H, m), 5.85-5.95 (1H, m), 7.23-7.4 (5H, m).

### D. Methyl 2-(2-cyclopentenyl)-3-phenyl-3-(benzyloxycarbonyl)amino-propionate

A solution of trans-3-(2-cyclopentenyl)-4-phenyl-1-(tert-butyldimethylsilyl)azetidin-2-one (9.18 gms, 28 mmoles) in 5% sulfuric acid in methanol was refluxed for 18 hours. At the end of this period the reaction mixture was cooled and carefully neutralized with powdered sodium bicarbonate. The contents of the flask were concentrated under vacuum and, after dilution with water (200 mL), were extracted with methylene chloride (3 x 150 mL). The combined organic layers were dried (anhydrous MgSO₄) and filtered. The solvents were removed under vacuum to afford hydrolyzed product as an orange tinted solid (6.61 gms, 100%) which was dissolved in ethyl acetate (70 mL) and cooled to 0°C. To it an aqueous potassium carbonate solution (2M, 67.4 mL, 135 mmole) and benzyl chloroformate (11.4 gms, 30 mmoles) were added. The reaction mixture was warmed to room temperature and the aqueous layer was extracted with additional ethyl acetate (2x100 mL). The combined organic layers were dried (anhydrous MgSO₄), filtered and concentrated under vacuum to afford methyl 2-(2-cyclopentenyl)-3-phenyl-3-(benzyloxycarbonyl)aminopropionate (11.6 gms, 100%).

¹H NMR (CDCl₃) δ 1.5-1.65 (1H, m), 1.85-2.05 (1H, m), 2.0-2.5 (2H, m), 2.66 (1H, dd J=10, 4 Hz), 3.0-3.15 (1H, m), 3.45 (3H, s), 5.1 (2H, bs), 5.87 (1H, s), 6.45 (1H, bd, J=9.4 Hz), 7.1-7.5 (10H, m).

### E. [1α,3α,4α,5α]-2-(Benzyloxycarbonyl)-3-phenyl-2-azabicyclo[3.3.0]octane-4-carboxylic acid, methyl ester

To a stirred solution of methyl 2-(2-cyclopentenyl)-3-phenyl-3-(benzyloxycarbonyl)aminopropionate (10.21 gms, 27 mmoles) in tetrahydrofuran (60 ml) at 0°C, a solution of mercuric triflouroacetate (16.0 gms, 38 mmoles) in tetrahydrofuran (65 mL) was added dropwise. The reaction mixture was warmed to room temperature and stirred for 15 minutes The reaction mixture was recooled to 0°C and to it a solution of sodium borohydride (4.1 gms, 108 mmoles) in water (40 mL) was added slowly; it was stirred for 30 minutes during which time grey precipitates were formed. At the end of this period the reaction mixture was filtered through diatomaceous earth (Celite (trademark)) and the filtrate was concentrated under vacuum. The residue was suspended in water and extracted with methylene chloride (3 x 100 ml). The combined organic layers were dried (anhydrous MgSO₄), filtered and concentrated under vacuum to afford [1α,3α,4α, 5α]-2-(benzyloxycarbonyl)-3-phenyl-2-azabicyclo[3.3.0]octane-4-carboxylic acid, methyl ester (10.9 gms, 100%) as a solid.

### F. [1α,3α,4α,5α]-2-(Benzyloxycarbonyl)-4-carboxamide-3-phenyl-2-azabicyclo[3.3.0]octane

To a suspension of the ammonium chloride (4.23 gm, 79 mmole) in benzene (40 ml) at 5°C, was slowly added a 2M solution (39.5 ml, 79 mmole) of trimethyl aluminum in hexane. After addition was complete, the reaction mixture was allowed to warm to room temperature and was stirred for 45 min. until gas evolution had ceased. To it a solution of [1α,3α,4α,5α]-2-(benzyloxycarbonyl)-3-phenyl-2-azabicyclo[3.3.0]octane-4-carboxylic acid, methyl ester (6.0 gm, 16 mmoles) in benzene (50 ml) was added and the solution was maintained at 50°C for 16 hours. The reaction mixture was cooled to room temperature and was carefully quenched with 5% HCl. Then the resulting mixture was filtered through diatomaceous earch (Celite (trademark)) and the residue was washed with methylene chloride (200 ml). The organic layer was separated while the aqueous layer was made basic and extracted with methylene chloride (200 ml). The organic extracts were combined, dried (anhydrous MgSO₄) and concentrated in vacuo to afford a residue which was suspended in 1:1 ether-pentane to afford [1α,3α,4α,5α]-2-benzyloxycarbonyl)-4-carboxamide-3-phenyl-2-azabicyclo[3.3.0]octane as white solid (2.91 gm, 50%).

¹H NMR δ (CDCl₃) 1.5-2.2 (6H, m), 2.9-3.2 (2H, m), 4.4-4.6 (1H, m), 4.7-5.4 (5H, m), 6.77 (1H, d, J=6.5 Hz), 7.0-7.4 (9H, m).

### G. [1α,3α,4α,5α]-4-Amino-2-(benzyloxycarbonyl)-3-phenyl-2-azabicyclo[3.3.0]octane

To a slurry of [1α,3α,4α,5α]-2-(benzyloxycarbonyl)-4-carboxamide-3-phenyl-2-azabicyclo[3.3.0]octane (2.84 gms, 7.8 mmoles) in acetonitrile (30 mL) and water (30 mL) was added bis[(trifluoroacetoxy)iodo]benzene. The reaction mixture was stirred for 2 days at room temperature and then poured into ether (200 mL). The ether layer was extracted with 1N HCl (2 x 100 mL). The combined acidic layers were made alkaline with 10% ammonium hydroxide and extracted with methylene chloride (3 x 100 ml). The combined methylene chloride layers were dried (anhydrous MgSO₄), filtered and concentrated under vacuum to afford [1α,3α,4α,5α]-4-amino-2-(benzyloxycarbonyl)-3-phenyl-2-azabicyclo[3.3.0]octane as an oil (1.25 gms, 47%).

¹H NMR (CDCl₃) δ 1.4-1.9 (1H, m), 1.95-2.1 (1H, m), 2.25-2.4 (1H, m), 3.2-3.4 (1H, m), 4.5-4.55 (1H, m), 4.7-5.1 (3H, m), 6.76 (1H, d, J=6.2 Hz), 7.0-7.4 (9H, m).

### H. [1α,3α,4α,5α]-4-(2-Methoxybenzyl)amino-3-phenyl-2-azabicyclo[3.3.0]octane

[1α,3α,4α,5α]-4-Amino-2-(benzyloxycarbonyl)-3-phenyl-2-azabicyclo[3.3.0]octane (0.532 gm, 1.6 mmole) was dissolved in methanol (20 ml) and the pH of the medium was adjusted to 5 with methanolic HCl. To it crushed 4A° molecular sieves ( 1.0 gm), sodium cyanoborohydride (0.102 gm, 1.6 mmole) and o-methoxy benzaldehyde (0.258 gm, 1.90 mmole) was added and the resulting reaction mixture was stirred at room temperature for 16 hours. At the end of this period the reaction mixture was filtered through diatomaceous earth (Celite (trademark)) and the filtrate was taken up in aqueous ammonium hydroxide. The aqueous phase was extracted with methylene chloride (3 x 60 ml) and dried (anhydrous MgSO₄). The solvents were removed under reduced pressure to afford an oily residue (0.813 gm). The residue was loaded on a short silica gel column. Elution with 30% ethyl acetate in hexane afforded product (0.617 g). The product was dissolved in ethanol (20 ml) and to it 10% palladium on carbon (1.2 gm) and ammonium formate (0.864 gm, 14 mmole) were added. The resulting reaction mixture was stirred at 25°C for 16 hours. At the end of this period the reaction mixture was filtered through diatomaceous earth (Celite (trademark)) which was washed with ethanol (50 ml) and methylene chloride (100 ml). The solvents were removed under vacuum to afford a solid which was taken up in aqueous ammonium hydroxide and extracted with methylene chloride (3 x 60 ml). The organic extracts were combined and dried (anhyd. MgSO₄). Evaporation of the solvents under reduced pressure afforded a yellow oil (441 mg) from which [1α,3α,4α,5α]-4-(2-methoxybenzyl)amino-3-phenyl-2-azabicyclo[3.3.0]octane (403 mg, 83%) was isolated as a white solid by treatment with ether-HCl. This was crystallized from ethanol/methanol to afford crystalline [1α,3α,4α,5α]-4-(2-methoxybenzyl)amino-3-phenyl-2-azabicyclo[3.3.0]octane hydrochloride (111 mg, 18%, m.p. 250°C). The structure was further confirmed by single crystal X-ray crystallographic analysis.

¹H NMR (CDCl₃) δ 1.2-1.55 (2H, m), 1.6-1.75 (1H, m), 1.8-2.1 (3H, m), 2.6 (1H, dd, J=16, 8Hz), 2.91 (1H, dd, J=4.5, 1.5 Hz), 3.51 (3H, s), 3.51 (1H, d, J=13.5 Hz), 3.75 (1H, d, J=13.5 Hz), 4.03 (1H, dd, J=13.6, 8 Hz), 4.27 (1H, d, J=4.5 Hz), 6.74 (1H, d, J=8 Hz), 6.84 (1H, dt, J=7.5 Hz), 7.03 (1H, d, J=7.5 Hz), 7.1-7.35 (6H, m).

¹³C-NMR (CDCl₃) δ 25.9, 31.5, 35.3, 47.2, 48.6, 54.8, 62.5, 64.3, 67.5, 109.0, 120.1, 126.7, 127.2, 128.0, 128.1, 128.3, 130, 139.1, 157.7.

### EXAMPLE 2

### 4-(2-Methoxybenzyl)amino-3-phenyl-2-azabicyclo[4.4.0]decane

### A. 3-Carboethoxy-2-phenylquinoline

Under a nitrogen atmosphere, in a round-bottom flask equipped with a pressure-equalizing addition funnel was placed 3.7 mL (47 mmol) of DMF, and the system was placed in an ice bath. To this stirring liquid was added dropwise 8.4 mL (89 mmol) of POCl₃. The mixture was warmed to 25°C using a warm water bath and stirred at room temperature for 0.5 hours. The system was cooled in an ice bath, and 12.0 g (44.9 mmol) of 2-carboethoxy-1-phenyl-1-(phenylamino)ethene in 45 mL of 1,1,2,2-tetrachloroethane was added dropwise to the system. The mixture was heated at 80°C for 3 hours, cooled and added slowly to iced water.
The mixture was made basic with saturated aqueous NaHCO₃ and extracted with two portions of CHCl₃. The combined organic fractions were washed with two portions of H₂O, dried (Na₂SO₄) and concentrated in vacuo to obtain 18.4 g of oil. This material was purified by flash column chromatography, gradually increasing the polarity of the eluant from hexanes to 8:1 hexanes/ethyl acetate, to obtain 5.2 g of the title compound.

¹H NMR (CDCl₃) δ 1.08 (t, 3H, J=8), 4.18 (q, 2H, J=8), 7.40-7.68 (m, 1H), 7.93 (d, 1H, J=10), 8.19 (d, 1H, J=10), 8.63 (s, 1H).

### B. 4-Carboethoxy-3-phenyl-2-azabicyclo[4.4.0]decane

3-Carboethoxy-2-phenylquinoline (1.5 g, 5.4 mmol), 150 mg of platinum oxide and 36 mL of glacial acetic acid were combined in a bottle and shaken (Parr apparatus) under an atmosphere of hydrogen (40 psi) for 1.5 hours. The mixture was diluted with ethanol and filtered through a pad of diatomaceous earth (Celite (trademark)), and the filtrate was concentrated in vacuo. The residue was made basic cautiously using saturated aqueous NaHCO₃, diluted with H₂O and extracted with two portions of CH₂Cl₂. The organic fractions were dried (Na₂SO₄) and concentrated with a rotary evaporator to obtain 1.4 g of oil. This material was subjected to flash column chromatography using 8:1 hexanes/ethyl acetate as the eluant to obtain 800 mg of the title compound as a solid, m.p. 70-73°C.

¹H NMR (CDCl₃) δ 1.02 (t, 3H, J=8), 1.15-1.80 (m, 8H), 1.88-1.98 (m, 1H), 2.15-2.28 (m, 3H), 2.85-2.92 (m, 1H), 3.08 (bs, 1H), 3.82-4.05 (m, 3H), 7.10-7.18 (m, 1H), 7.25-7.42 (m, 4H).

### C. 2-Carbobenzyloxy-4-carboethoxy-3-phenyl-2-azabicyclo[4.4.0]decane

In a round-bottom flask were placed 900 mg (3.1 mmol) of the title compound from part B above and 7 mL of 10% aqueous NaHCO₃. To this stirring mixture at 0°C was added 0.49 mL (3.4 mmol) of benzyl chloroformate, and the mixture was stirred at 0°C for 1 hour. The mixture was extracted with two portions of ethyl ether, and the ethyl ether fractions were washed with brine, dried (Na₂SO₄) and concentrated with a rotary evaporator to obtain 1.1 g of oil. This material was purified by flash column chromatography to obtain 950 mg of the title compound.

¹H NMR (CDCl₃) δ 0.80-1.75 (m, 11H), 1.88-2.00 (m, 2H), 2.37-2.45 (m, 1H), 2.90-3.00 (m, 1H), 3.98-4.43 (m, 3H), 5.18-5.38 (m, 2H), 6.00 - 6.15 (2d, 1H, J=6), 7.13-7.55 (m, 10H).

### D. 2-Carbobenzyloxy-4-carboxamido-3-phenyl-2-azabicyclo[4.4.0]decane

Under a nitrogen atmosphere in a round-bottom flask were placed 572 mg (10.7 mmol) of ammonium chloride and 8 mL of benzene. To this system was added 5.4 ml of 2M trimethylaluminum in hexanes. To this stirring mixture at 0°C was added 900 mg (2.30 mmol) of the title compound from part C above in 12 mL of benzene, and the reaction mixture was heated at 50°C overnight. The system was cooled in an ice bath, the reaction was slowly quenched with 8 mL of 1M aqueous HCl, and the mixture was stirred for 0.5 hours. The mixture was filtered through diatomaceous earth (Celite (trademark)) and the filter pad was washed with saturated aqueous NaHCO₃ and CH₂Cl₂. The layers were separated, the aqueous phase was extracted with CHCl₂ and the combined organic fractions were dried (Na₂SO₄) and concentrated in vacuo. The crude material was purified by flash column chromatography using 1:49 methanol/CHCl₃ as the eluant to obtain 450 mg of the title compound.

¹H NMR (CDCl₃) δ 0.72-2.00 (m, 10H), 2.28-2.45 (m, 1H), 2.82-2.90 (m, 1H), 4.18-4.43 (m, 1H), 5.15-5.85 (m, 4H), 5.88 - 5.98 (2d, 1H, J=6), 7.18-7.52 (m, 10H).

### E. 4-Amino-2-carbobenzyloxy-3-phenyl-2-azabicylo[4.4.0]decane

In a round-bottom flask were placed 400 mg (1.02 mmol) of the title compound from part D above and 2 mL of CH₃CN. To the system were added 2 mL of H₂O and 639 mg (1.48 mmol) of bis-(trifluoroacetoxy)iodobenzene. The reaction mixture was stirred at room temperature overnight. The reaction mixture was partitioned between ether and 1M aqueous HCl, the layers were separated and the ether phase was extracted with 1M aqueous HCl. The combined aqueous fractions were neutralized with saturated aqueous NaHCO₃ and extracted with two portions of ether. These combined ether extracts were washed with brine, dried (Na₂SO₄) and concentrated with a rotary evaporator to obtain 200 mg of the title compound.

¹H NMR (CDCl₃) δ 1.00-1.72 (m, 11H), 2.02-2.15 (m, 1H), 2.22-2.45 (m, 1H), 3.28-3.40 (m, 1H), 4.10-4.35 (m, 1H), 5.21 (d, 2H, J=6), 5.35-5.66 (m, 1H), 7.15-7.80 (m, 10H).

### F. 2-Carbobenzyloxy-4-(2-methoxybenzyl)amino-3-phenyl-2-azabicyclo[4.4.0]decane

Under a nitrogen atmosphere in a round-bottom flask were placed 200 mg (0.55 mmol) of the title compound from part E above and 2 mL of methanol. To the system was added ca. 0.5 g of 3Å molecular sieves, and the pH of the mixture was adjusted to ca. 4 using methanol saturated with HCl. To the system was added 38 mg (0.60 mmol) of sodium cyanoborohydride in portions, and the pH of the system was adjusted to ca. 4 as above. To the system was added 89 mg (0.66 mmol) of o-anisaldehyde, and the reaction mixture was stirred at room temperature overnight.

The reaction mixture was made basic using saturated aqueous sodium bicarbonate and filtered through a pad of diatomaceous earth (Celite(trademark)). The pad was rinsed with methanol and CH₂Cl₂. The filtrate was concentrated in vacuo, and the residue was partitioned between CH₂Cl₂ and H₂O. The layers were separated, the aqueous phase was extracted with CH₂Cl₂ and the combined organic fractions were dried (Na₂SO₄) and concentrated with a rotary evaporator to obtain 220 mg of oil. The crude material was purified by flash column chromatography to obtain 110 mg of the title compound.

¹H NMR (CDCl₃) δ 0.98-2.04 (m, 11H), 2.20-2.38 (m, 1H), 2.98-3.16 (m, 1H), 3.66 (s, 3H), 3.77 (s, 2H), 4.10-4.32 (m, 1H), 5.10-5.24 (m, 2H), 5.54-5.74 (2bs, 1H), 6.70-6.88 (m, 2H), 7.00-7.40 (m, 10H), 7.66-7.84 (m, 2H).

### G. 4-(2-Methoxybenzyl)amino-3-phenyl-2-azabicyclo[4.4.0]decane hydrochloride

Under a nitrogen atmosphere in a round-bottom flask were placed 110 mg (0.23 mmol) of the title compound from part F above and 1.5 mL of ethanol. To the system were added 72 mg (1.2 mmol) of ammonium formate and 110 mg of 10% Pd/C, and the mixture was stirred at room temperature for 3 hours. The reaction mixture was filtered through a pad of diatomaceous earth, and the pad was rinsed with ethanol and CH₂Cl₂. The filtrate was concentrated, and the residue was partitioned between CH₂Cl₂ and saturated aqueous NaHCO₃. The layers were separated and the aqueous phase was extracted with CH₂Cl₂. The combined organic fractions were dried (Na₂SO₄) and concentrated to obtain 75 mg of oil. This material was dissolved in CH₂Cl₂, and the solution was extracted with two portions of 1M aqueous HCl. The combined aqueous extracts were washed with CH₂Cl₂ and made basic with 1M aqueous NaOH. The aqueous fraction was extracted with two portions of CH₂Cl₂, and these combined extracts were dried (Na₂SO₄) and concentrated with a rotary evaporator to obtain 63 mg of oil. This material was diluted with CH₂Cl₂, and ether saturated with HCl was added to the system. Concentration and trituration with ether afforded 60 mg of the title compound as its HCl salt, mp 195-198°C.

¹H NMR (CDCl₃) δ 1.42-2.04 (m, 12H), 2.34-2.48 (m, 1H), 2.72-2.80 (m, 1H), 2.98-3.04 (m, 1H), 3.38 (d, 1H, J=12), 3.42 (s, 3H), 3.58 (d, 1H, J=12), 3.95 (d, 1H, J=3), 6.66 (d, 1H, J=8), 6.80 (t, 1H, J=6), 6.97 (d, 1H, J=8), 7.10-7.30 (m, 6H).

### EXAMPLE 3

### 3-(2-Methoxybenzyl)amino-2-phenyl-1,2,3,4-tetrahydro-quinoline

In a bottle were placed 800 mg (2.9 mmol) of 3-carboethoxy-2-phenylquinoline, 0.174 mL (3.0 mmol) of acetic acid, 80 mg of PtO₂ and 48 mL of ethanol, and the mixture was shaken under an atmosphere (40 psi) of hydrogen (Parr apparatus) for 6 hours at room temperature. The mixture was filtered through diatomaceous earth (Celite(trademark)), and the filter pad was rinsed with ethanol. The filtrate was concentrated in vacuo. The residue was partitioned between CH₂Cl₂ and saturated aqueous sodium bicarbonate, the layers were separated, the aqueous phase was extracted with CH₂Cl₂ and the combined organic fractions were dried (Na₂SO₄) and concentrated. The crude material was purified by flash column chromatography to obtain 260 mg of 3-carboethoxy-2-phenyl-1,2,3,4-tetrahydroquinoline.

¹H NMR (CDCl₃) δ 1.20 (t, 3H, J=6), 2.85-2.95 (m, 2H), 3.22-3.30 (m, 1H), 4.08-4.18 (m, 2H), 4.42 (bs, 1H), 4.95 (d, 1H, J=4), 6.60 (d, 1H, J=10), 6.70 (t, 1H, J=8), 7.02-7.34 (m, 7H).

This material was converted to the title compound using procedures analogous to those of Examples 2B-2G.

¹H NMR (CDCl₃) δ 2.62-2.74 (m, 1H), 2.84-2.94 (m, 1H), 3.12-3.21 (m, 1H), 3.59 (s, 3H) , 3.74 (d, 1H, J=9), 3.82 (d, 1H, J=9), 4.61 (d, 1H, J=3), 6.50-6.82 (m, 4H), 6.92-7.36 (m, 9H).

### EXAMPLE 4

### Trans-4-(2-methoxybenzylamino)-2-methyl-3-phenyl-3,4-dihydro-1(2H)-isoquinolinone

A solution of trans-4-amino-2-methyl-3-phenyl-3,4-dihydro-1(2H)-isoquinolinone (2.36 g, 9.36 mmol), prepared according to the method of I. Attanassova et al., Commun. Dept. Chem., Bulg. Acad. Sci., 17(2), 172-9 (1984), in 40 mL acetic acid was treated with 1.5 g of 3Å molecular sieves (Aldrich Chemical Co.) followed by the addition of 1.59 g (11.7 mol) of ortho-anisaldehyde. Sodium triacetoxyborohydride (Aldrich) was added in three 1.32 g portions (total of 3.97 g, 18.7 mmol), and then stirred at 25 °C overnight. The sieves were removed by filtration through a pad of diatomaceous earth (Celite (trademark)), the pad being washed with additional acetic acid, and the combined filtrates were concentrated in vacuo. The residue was treated with 30 mL H₂O, acidified with 1 N HCl and extracted with diethyl ether. The aqueous layer was made alkaline with 2 N NaOH and was reextracted with diethyl ether. After drying over MgSO₄, the ether was removed in vacuo to give the crude title compound as an oil (1.65 g). Chromatography on silica gel (230-400 mesh) eluting with methylene chloride:methanol:concentrated ammonium hydroxide (97:2:1) gave the pure trans isomer of the title compound as a clear oil (0.80g, 23%).

MS, m/e 372 (M+), 253, 132, 121.

¹H NMR (300 MHz, CDCl₃, δ) 3.14 (s, 3H), 3.78 (s, 3H), 3.72-3.92 (m, 3H), 4.88 (bs, 1H), 6.82-6.98 (m, 4H), 7.10-7.42 (m, 9H), 8.18 (d, 1H).

### EXAMPLE 5

### Trans-4-(2-methoxybenzylamino)-3-phenyl-3,4-dihydro-1(2H)-isoquinolinone

Using the procedure described in Example 4, trans-4-amino-3-phenyl-3,4-dihydro-1(2H)-isoquinolinone (which was prepared by the method of I. Attanassova et al., Commun. Dept. Chem., Bulg. Acad. Sci., 17 (2), 179-9 (1984) except that N-(trimethylsilyl)benzylidenamine was substituted for N-benzylidenemethylamine in the first step of the synthesis), was immediately reacted with ortho-anisaldehyde to produce the title compound as a pale yellow viscous oil (0.100 g).

MS, m/e 358 (M+), 253, 132, 121.

¹H NMR (CDCl₃, 300 MHz, δ) 3.74 (s, 3H), 3.78-4.00 (m, 3H), 4.94 (t, 1H), 6.16 (bs, 1H), 6.82 (d, 1H), 6.92 (t, 1H), 7.12-7.50 (m, 10H), 8.15 (d, 1H).

### EXAMPLE 6

### Trans-4-(2-methoxybenzylamino)-2-methyl-3-phenyl-1,2,3,4-tetrahydroisoquinoline

The title compound from Example 4 (0.8 g, 2.15 mmol) was dissolved in 10 mL dry THF under nitrogen and treated with lithium aluminum hydride (LAH, 0.4 g, 10.5 mmol). After 3 hours the excess LAH was quenched with water and extracted with ethyl acetate. The organic extracts were dried (MgSO₄) and concentrated to an oil (0.60 g). Chromatography on silica gel (230-400 mesh) eluting with ethyl acetate:hexane (1:1) gave the title compound (R_{f} 0.17) as a viscous oil.

MS, m/e 359 (M⁺¹), 358 (M⁺), 239, 222, 121.

¹H NMR (CDCl₃, 300 MHz, δ) 2.23 (s, 3H), 3.50-3.99 (m, 9H) 4.12 (d, 1H), 6.74-6.96 (m, 4H), 7.02-7.70 (m, 9H).

### EXAMPLE 7

### Trans-4-(2-methoxybenzylamino)-3-phenyl-1,2,3,4-tetrahy-droisoquinoline dihydrochloride

Using the procedure described in Example 6, the title compound from Example 5 (0.25 g, 0.7 mmol) was reduced with 0.13 g LAH in 10 mL THF to give, after chromatography on silica gel (230-400 mesh) eluting with methylene chloride:methanol:concentrated ammonium hydroxide (95:4:1), a light brown oil. The oil was treated with gaseous hydrochloric acid in diethyl ether to provide pure trans-4-(2-methoxybenzylamino)-3-phenyl-1,2,3,4-tetrahydroisoquinoline dihydrochloride, mp 138-144 °C. Anal. for C₂₃H₂₄N₂O·2HCl·H₂O: C 63.45, H 6.48, N 6.43. Found C 63.09, H 6.59, N 6.27.

¹H NMR (free base, CDCl₃, 300 MHz, δ) 3.54-4.18 (m, 9H), 6.78-6.86 (m, 2H), 7.0 (m, 1H), 7.09-7.44 (m, 10H).

### EXAMPLE 8

### 5-(2-Methoxybenzyl)amino-4-benzhydryl-3-azabicyclo-[4.1.0]heptanes

### A. 5-Hydroxy-4-benzhydryl-3-azabicyclo[4.1.0]-heptanes

To a stirred solution of 2,2-diphenyl-nitroethane (4.21 g, 18.5 mmole) and methyl cis-2-(formyl)cyclopropanecarboxylate (2.61 g, 20.4 mmol) in methylene chloride (50mL) was added basic alumina (20.0 g). The reaction mixture was then stirred at 25°C for 24 hours. At the end of this period, it was filtered, and the alumina was washed with 50% ethyl acetate in hexane. The combined organic solvents were concentrated to afford yellow a oil (3.49 g) as a mixture of isomers which was used without purification. This was dissolved in ethanol (65 ml) at 25°C, and to it neutral Raney nickel was added (3.5 g). The reaction mixture was shaken on a Parr shaker under hydrogen (45 psi) for 4 hours; thereafter the reaction mixture was filtered through diatomaceous earth (Celite (trademark)) which was washed thoroughly with ethanol (100 ml), and methylene chloride (100 ml). The organic phases were combined and concentrated under vacuum to a yellow oil (2.65 g) which was loaded onto a silica gel column. Elution with 95:5:0.5 methylene chloride:methanol:aqueous ammonia afforded 5-hydroxy-4-benzhydryl-3-azabicyclo[4.1.0]heptanes (0.291 g, mp 250°C, 6%).

IR (film) Vₘₐₓ 3500, 1630, 1650 cm⁻¹

¹H NMR (CDCl₃) δ 1.12-1.3 (2H, m), 1.7-1.8 (2H, m), 3.8 (1H, dd, J = 1.5, 12 Hz), 3.87 (1H, bs), 4.22 (1H, d, J = 11 Hz), 5.07 (1H, bs), 7.14-7.4 (1OH,m).

### B. 5-Oxo-4-benzhydryl-3-azabicyclo[4.1.0]heptanes

To a solution of oxalyl chloride (140 mgs, 1.15 mmole) in methylene chloride (3 ml) at -50°C was added dimethyl sulfoxide (180 mgs, 2.3 mmole). To it a solution of 5-hydroxy-4-benzhydryl-3-azabicyclo[4.1.0]heptane (287 mgs, 1.04 mmole) in methylene chloride (4ml) was added and the reaction mixture was further stirred for 15 minutes at 50°C and for 5 minutes at 0°C. Thereafter, triethylamine (0.7 ml) was added, and the reaction mixture was stirred at 25°C for 3 hours. At the end of this period, the reaction mixture was taken up in ether; the organic layer was washed with water and dried (anhyd. MgSO₄). The organic phases were removed under vacuum to afford 5-oxo-4-benzhydryl-3-azabicyclo[4.1.0]heptanes (275 mgs).

¹H NMR (CDCl₃) δ 0.45 (0.6H, q, J = 5 Hz), 1.05-1.3 (1.4H, m), 2.0-2.3 (1.4H, m), 2.65-2.85 (0.6H, m), 4.55 (0.4H, d, J = 6Hz), 4.7 (0.6H, d, J = 6Hz), 4.75 (0.4H, d, J = 6Hz), 4.82 (0.6H, bd), 5.4 (0.4H, bs), 5.55 (0.6H, bs), 7.1-7.4 (10H, m).

### C. 5-(2-Methoxybenzyl)amino-4-benzhydryl-3-azabicyclo[4.1.0]heptanes

A solution of 5-oxo-4-benzhydryl-3-azabicyclo[4.1.0]heptanes (275 mgs), hydroxyl amine hydrochloride (210 mgs, 3 mmole) and sodium acetate (410 mgs, 5 mmole) in ethanol (15 ml) containing water (2 ml) was stirred at 25°C for 18 hours. At the end of this period, the reaction mixture was concentrated under vacuo and was suspended in water. This, after extractive work up with methylene chloride, afforded 5-oxamino-4-benzhydryl-3-azabicyclo[4.1.0]heptanes (275 mgs). This was used in the next without purification. Thus, to a stirred solution of 5-oxamino-4-benzhydryl-3-azabicyclo[4.1.0]heptanes (275 mgs) in ethanol (10 ml) was added neutral Raney Ni (∼0.5 gms) and the Parr bottle was shaken under hydrogen (40 psi). After 4 hours, the reaction mixture was filtered through diatomaceous earth (Celite (trademark)) which was thoroughly washed with ethanol. The organic solvents were removed under vacuum to afford 5-amino-4-benzhydryl-3-azabicyclo[4.1.0]heptane which was converted to the title compound by a procedure similar to that described in Examples 1H and 7.

¹H NMR (CDCl₃) δ 0.3-0.8 (2H, m), 1.0-1.4 (2H, m), 2.6-3.5 (4H, m), 3.7, 3.9 (3H, s), 4.20 (0.4H, d, J = 11 Hz), 4.39, (0.6H, d, J = 11 Hz), 6.7-7.5 (104H, m). HRMS Calculated for C₂₇H₃₀N₂O: 398.2353. Found: 398.2333.

The title compounds of Examples 9 through 14 were prepared by a procedure similar to that described in Example 1.

### EXAMPLE 9

### [1α,3α,4β,5α]-4-(2-chlorobenzyl)amino-3-phenyl-2-azabicyclo[3.3.0]octane

¹H NMR (CDCl₃) δ 1.4-2.1 (6H, m), 2.73 (1H, quin, J = 7.5 Hz), 3.12 (1H, dd, J = 7.5, 9.5 Hz), 3.55 (1H, dd, J = 13 Hz), 3.72 (1H, dd, J = 13 Hz), 3.8 (1H, d, J = 13.5 Hz), 3.93 (1H, m), 7.0-7.4 (9H, m).

¹³C-NMR (CDCl₃) δ 25.7, 26.4, 37.4, 45.5, 52.6, 62.3, 66.0, 68.1, 126.8, 127.4, 127.5, 128, 128.3, 128.5, 140.5, 142.0.

HRMS Calculated for C₂₀H₂₃N₂Cl: 326.1544. Found: 326.1481.

### EXAMPLE 10

### [1α,3α,4β,5α]-4-(2-methoxybenzyl)amino-3-phenyl-2-azabicyclo[3.3.0]octane

¹H NMR (CDCl₃ δ 1.4-2.1 (6H, m), 2.69 (1H, quin, J=7.5 Hz), 2.96 (1H, dd, J=7.5, 9.5 Hz), 3.53 (3H, s), 3.56 (1H, dd, J=13.5 Hz), 3.74 (1H, dd, J=13.5 Hz), 3.56 (1H, d, J=9.5 Hz), 3.86-3.92 (1H, m), 6.74 (1H, d, J=8.2 Hz), 6.83 (1H, dt, J=7.0, 1.0 Hz), 7.05 (1H, dd, J=7.5, 2.0 Hz), 7.1-7.4 (6H, m).

HRMS Calc'd for C₂₁H₂₆N₂O:322.2045. Found: 322.2056.

Anal. calc'd for C₂₁H₂₆N₂O·2HCl·1/4H₂O: C, 63.08; H, 7.18; N, 7.01 Found: C, 62.89; H, 6.96; N, 7.01.

### EXAMPLE 11

### [1α,3α,4α,5α]-4-(2,5-Dimethoxybenzyl)amino-3-phenyl-2-azabicyclo[3.3.0]octane

¹H NMR (CDCl₃) δ 1.2-1.45 (2H, m), 1.5-1.8 (1H, m), 1.8-2.0 (3H, m), 2.59 (1H, dd, J=15.4, 7 Hz), 2.92 (1H, dd, J=4.5, 1.5 Hz), 3.49 (3H, s), 3.48 (1H, d, J=13.5 Hz), 3.71 (1H, d, J=13.5 Hz), 3.72 (3H, s), 3.95-4.05 (1H, m), 4.28 (1H, d, J=4.5 Hz), 6.6-6.75 (3H, m), 7.2-7.4 (5H, m).

¹³C NMR (CDCl₃) δ 25.9, 31.5, 35.2, 47.2, 48.6, 55.3, 55.7, 62.5, 64.3, 67.5, 110.7, 112.2, 116.1, 126.8, 127.2, 128.3, 129.1, 139.1, 151.9, 153.2.

### EXAMPLE 12

### [1α,3α,4α,5α]-4-(2-Methoxy-5-chlorobenzyl)amino-3-phenyl-2-azabicyclo[3.3.0]octane

¹H NMR (CDCl₃) δ 1.25-1.55 (3H, m), 1.6-1.7 (1H, m), 1.8-2.05 (2H, m), 2.63 (1H, dd, J=17, 8.5 Hz), 2.91 (1H, dd, J=4.6, 1.5 Hz), 3.51 (3H, s), 3.54 (1H, d, J=13.5 Hz), 3.78 (1H, d, J=13.5 Hz), 3.95-4.05 (1H, m), 4.28 (1H, d, J=4.5 Hz), 6.74 (1H, d, J=8.2 Hz), 6.84 (1H, dt, J=7.4, 1Hz), 7.03 (1H, dd, J=7.3, 1.7 Hz), 7.15-7.4 (5H, m).

¹³C NMR(CDCl₃) δ 25.9, 31.5, 35.1, 47.2, 48.4, 54.8, 62.6, 64.2, 67.4, 109.9, 120.2, 126.8, 127.2, 127.5, 128.3, 128.4, 130.1, 138.7, 157.7.

### EXAMPLE 13

### [1α,3α,4α,5α]-4-(2-Methoxy-5-methylbenzyl)amino-3-phenyl-2-azabicyclo[3.3.0]octane

¹H NMR (CDCl₃) δ 1.25-1.45 (3H, m), 1.6-1.75 (1H, m), 1.85-2.05 (2H, m), 2.23 (3H, s), 2.61 (1H, dd, J=16.8, 8.6 Hz), 2.91 (1H, dd, J=5, 1.5 Hz), 3.49 (3H, s), 3.49 (1H, d, J=13.5 Hz), 3.7 (1H, d, J=13.5 Hz), 3.95-4.05 (1H, m), 4.27 (1H, d, J=4.5 hz), 6.62 (1H, d, J=8.3 Hz), 6.82 (1H, d, J=2 Hz), 6.9 (1H, dd, J=8.2, 1.8 Hz), 7.15-7.35 (5H, m).

¹³C NMR (CDCl₃) δ 20.4, 25.9, 31.5, 35.2, 47.2, 48.6, 54.9, 62.5, 64.3, 67.6, 109.9, 126.7, 127.2, 127.7, 128.2, 128.3, 130.7, 139.1, 155.6.

HRMS Calc'd for C₂₂H₂₈N₂O: 336.4760. Found: 336.2200.

### EXAMPLE 14

### [1α,3α,4α,5α]-4-(2 -Methoxy-5-fluorobenzyl)amino-3-phenyl-2-azabicyclo[3.3.0]octane

¹H NMR (CDCl₃) δ 1.3-1.5 (3H, m), 1.6-1.75 (1H, m), 1.8-2.0 (2H, m), 2.6 (1H, dd, J=17, 8.5 Hz), 2.91 (1H, dd, J=4.6, 1.7 Hz), 3.48 (1H, d, J=14 Hz), 3.51 (3H, s), 3.69 (1H, d, J=14 Hz), 3.9-4.1 (1H, m), 4.31 (1H, d, J=4.5 Hz), 6.64 (1H, dd, J=9, 4.3 Hz), 6.77 (1H, dd, J=8.8, 3.1 Hz), 6.85 (1H, dd, J=8.6, 3.2 hz), 7.2-7.5 (5H, m).

¹³C NMR (CDCl₃) δ 25.9, 31.4, 46.7, 48.6, 55.4, 62.5, 64.3, 67.4, 110.5, 110.6, 113.4, 113.7, 116.4, 116.7, 127, 127.2, 128.4, 138.6, 153.6, 158.3.

HRMS Calc'd for C₂₁H₂₅N₂OF: 340.4393. Found: 340.1944.

## Claims (Claims for the following Contracting State(s): AT, BE, CH, DE, DK, FR, GB, IT, LI, LU, NL, SE)

1. A compound of the formula 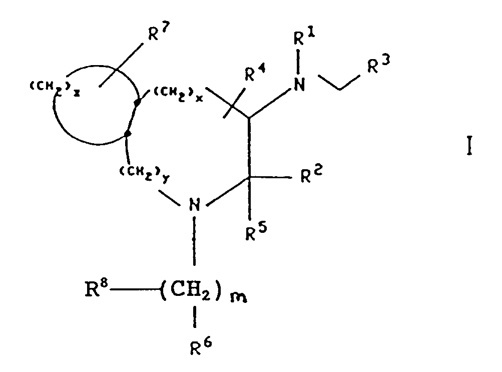 wherein x is an integer from zero to four;
y is an integer from zero to four;
z is an integer from one to six;
the ring containing (CH₂)_{z} may contain from zero to three double bonds, and one of the carbons of (CH₂)_{z} may optionally be replaced by oxygen, sulfur or nitrogen;
m is an integer from zero to twelve, and any one of the carbon-carbon single bonds of (CH₂)ₘ may optionally be replaced by a carbon-carbon double or triple bond and any one of the carbon atoms of said (CH₂)ₘ may optionally be substituted with R⁸;
R¹ is hydrogen or (C₁-C₆)alkyl optionally substituted with hydroxy, alkoxy or fluoro;
R² is a radical selected from
aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl groups may optionally be substituted with one or more substituents independently selected from halo, nitro, (C₁-C₆)alkyl, (C₁-C₆) alkoxy, trifluoromethyl, amino, (C₁-C₆)-alkylamino, 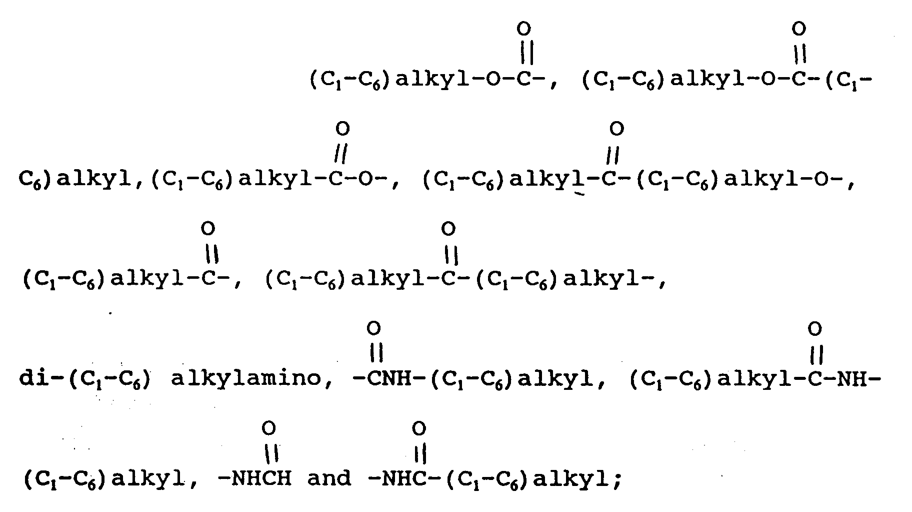 R⁵ is hydrogen or (C₁-C₆)alkyl;
or R² and R⁵, together with the carbon to which they are attached, form a saturated carbocyclic ring having from 3 to 7 carbon atoms, wherein one of said carbon atoms may optionally be replaced by oxygen, nitrogen or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; or cycloalkyl having from three to seven carbon atoms, wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, said substitutents being independently selected from halo, nitro, (C₁-C₆) alkyl, (C₁-C₆) alkoxy, trifluoromethyl, amino, phenyl, (C₁-C₆)-alkylamino, 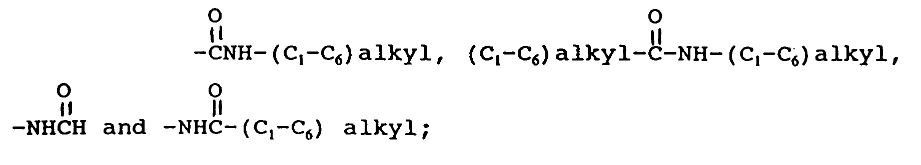 R⁴ may be attached to any atom of the nitrogen containing ring having an available bonding site and R⁷ may be attached to any atom of the (CH₂)_{z} containing ring having an available bonding site;
R⁴, R⁶, R⁷ and R⁸ are each independently selected from hydrogen, hydroxy, halo, amino, carboxy, carboxyalkyl, (C₁-C₆)alkylamino, di-(C₁-C₆)alkylamino, (C₁-C₆)alkoxy, 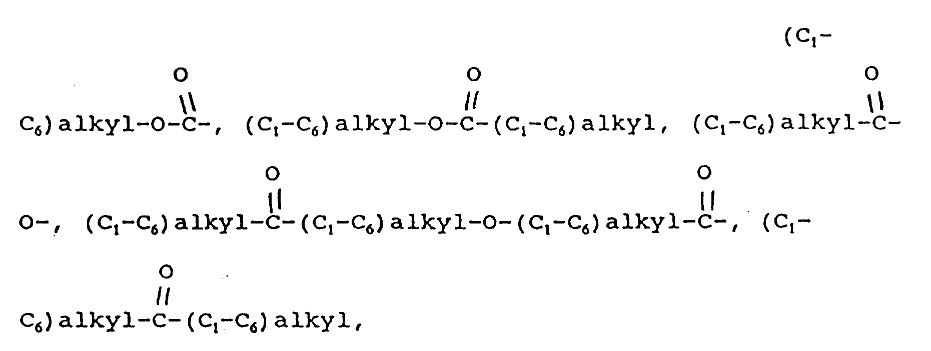 and the radicals set forth in the definition of R²;
or a pharmaceutically acceptable salt thereof.

2. A compound according to claim 1, wherein R¹, R⁴, R⁵, R⁶, R⁷ and R⁸ are hydrogen; R² is phenyl; R³ is methoxyphenyl wherein the phenyl moiety is substituted with hydrogen, chlorine, fluorine, (C₁-C₆)alkoxy or trifluoromethane; m is zero; and each of x, and y is an integer from zero to four, and z is an integer from one to four.

3. A compound according to claim 1, wherein said compound is [1α,3α,4α,5α]-4-(2-methoxybenzyl)amino-3-phenyl-2-azabicyclo[3.3.0]octane.

4. A compound according to claim 1, wherein said compound is 4-(2-methoxybenzyl)amino-3-phenyl-2-azabicyclo[4.4.0]decane.

5. A compound according to claim 1, wherein said compound is 5-(2-methoxybenzyl)amino-4-benzhydryl-3-azabicyclo[4.1.0]heptane.

6. A compound according to claim 1, wherein said compound is 3-(2-methoxybenzyl)amino-2-phenyl-1,2,3,4-tetrahydroquinoline.

7. A compound according to claim 1, wherein said compound is trans-4-(2-methoxybenzylamino)-2-methyl-3-phenyl-3,4-dihydro-1(2H)-isoquinolinone.

8. A compound according to claim 1, wherein said compound is trans-4-(2-methoxybenzylamino)-3-phenyl-3,4-dihydro-1(2H)-isoquinolinone.

9. A compound according to claim 1, wherein said compound is trans-4-(2-methoxybenzylamino)-2-methyl-3-phenyl-1,2,3,4-tetrahydroisoquinoline.

10. A compound according to claim 1, wherein said compound is trans-4-(2-methoxybenzylamino)-3-phenyl-1,2,3,4-tetrahydroisoquinoline dihydrochloride.

11. A compound according to claim 1, wherein said compound is 5-(2-methoxybenzyl)amino-4-benzhydryl-3-azabicyclo[4.4.0]decane;

12. A compound according to claim 1, wherein said compound is [1α,3α,4β,5α]-4-(2-methoxybenzyl)amino-3-phenyl-2-azabicyclo[3.3.0]octane.

13. A compound according to claim 1, wherein said compound is [1α,3α,4β,5α]-4-(2-chlorobenzyl)amino-3-phenyl-2-azabicyclo[3.3.0]octane.

14. A compound according to claim 1, wherein said compound is [1α,3α,4α,5α]-4-(2,5-dimethoxy- benzyl)amino-3-phenyl-2-azabicyclo[3.3.0]octaine.

15. A compound according to claim 1, wherein said compound is [1α,3α,4α,5α]-4-(2-methoxy-5-chlorobenzyl)amino-3-phenyl-2-azabicyyclo[3.3.0]octane.

16. A compound according to claim 1, wherein said compound is [1α,3α,4α,5α]-4-(2-methoxy-5-methylbenzyl)amino-3-phenyl-2-azabicyclo[3.3.0]octane.

17. A compound of the formula 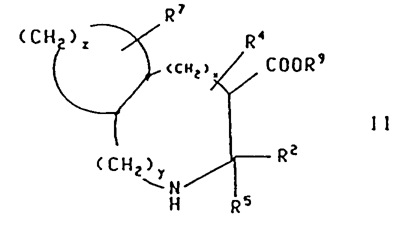 wherein R², R⁴, R⁵, R⁷, x, y and z are defined as in claim 1, and R⁹ is (C₁-C₆)alkyl or hydrogen.

18. A phamaceutical composition comprising a compound according to claim 1 and a phamaceutically acceptable carrier.

## Claims (Claims for the following Contracting State(s): ES, GR)

1. A process for preparing a compound of the formula 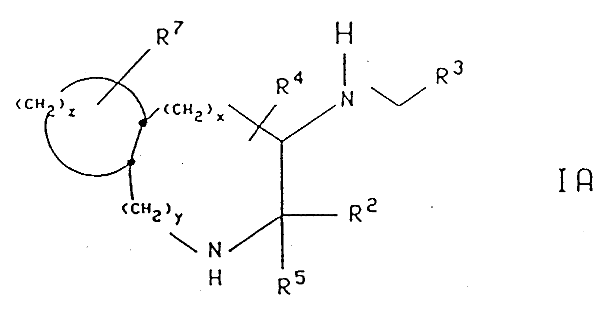 wherein x is an integer from zero to four;
y is an integer from zero to four;
z is an integer from one to six;
the ring containing (CH₂)_{z} may contain from zero to three double bonds, and one of the carbons of (CH₂)_{z} may optionally be replaced by oxygen, sulfur or nitrogen;
R² is a radical selected from
aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl groups may optionally be substituted with one or more substituents independently selected from halo, nitro, (C₁-C₆)alkyl, (C₁-C₆) alkoxy, trifluoromethyl, amino, (C₁-C₆)-alkylamino, 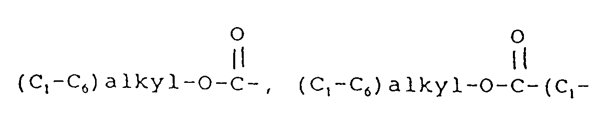 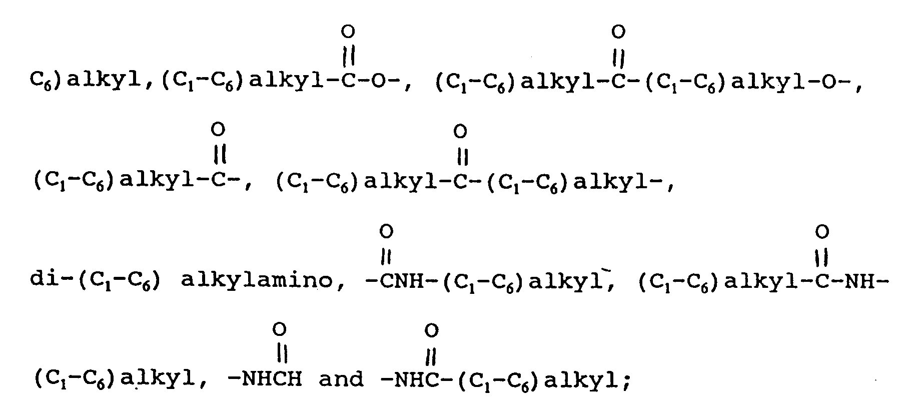 R⁵ is hydrogen or (C₁-C₆)alkyl;
or R² and R⁵, together with the carbon to which they are attached, form a saturated carbocyclic ring having from 3 to 7 carbon atoms, wherein one of said carbon atoms may optionally be replaced by oxygen, nitrogen, or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; or cycloalkyl having from three to seven carbon atoms, wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, said substitutents being independently selected from halo, nitro, (C₁-C₆) alkyl, (C₁-C₆) alkoxy, trifluoromethyl, amino, phenyl, (C₁-C₆)-alkylamino, 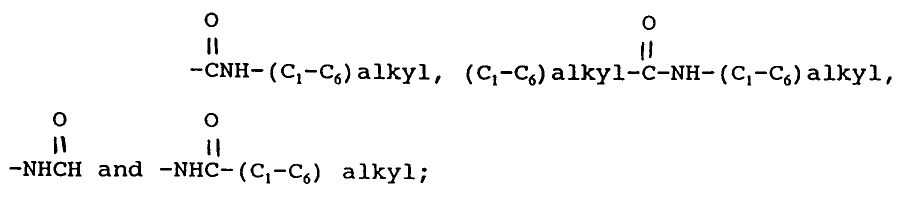 R⁴ may be attached to any atom of the nitrogen containing ring having an available bonding site and R⁷ may be attached to any atom of the (CH₂)_{z} containing ring having an available bonding site;
R⁴, and R⁷ are each independently selected from hydrogen, hydroxy, halo, amino, carboxy, carboxyalkyl, (C₁-C₆)alkylamino, di-(C₁-C₆)alkylamino, (C₁-C₆)alkoxy, 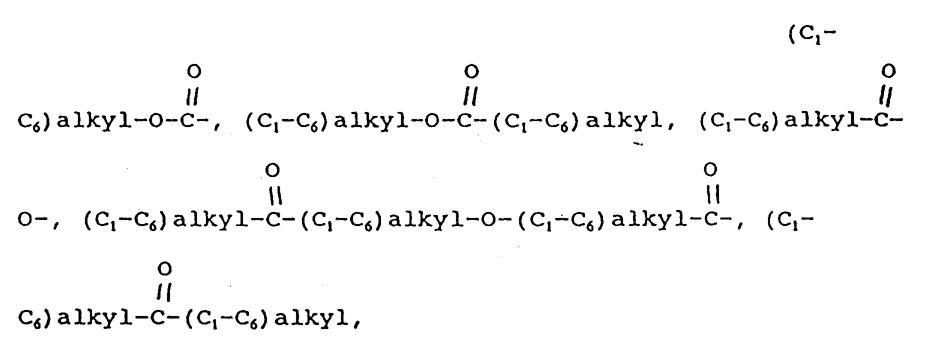 and the radicals set forth in the definition of R²;
or a pharmaceutically acceptable salt thereof,
comprising reacting a compound of the formula 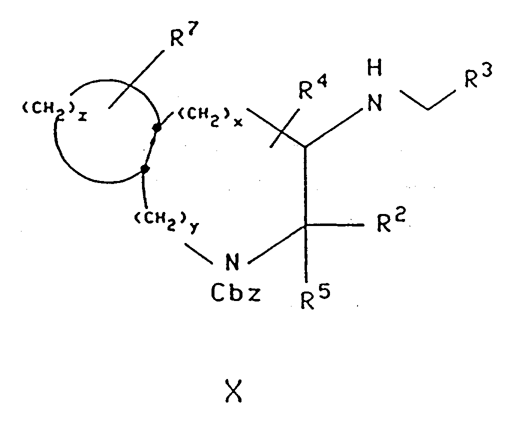 wherein R², R³, R⁴, R⁵, R⁷, x, y and z are defined as above and cbz is carbobenzyloxy, with ammonium formate, in the presence of palladium on charcoal.

2. A process according to claim 1, wherein said compound of formula X is obtained by either:
(a) reductive amination of a compound of the formula R³CHO, wherein R³ is defined as in claim 1, in the presence of a compound of the formula 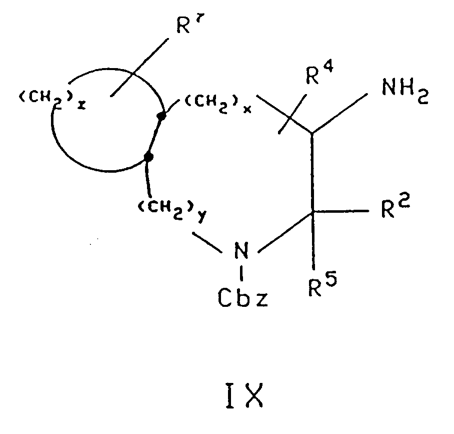 wherein R², R³, R⁴, R⁵, R⁷, x, y and z are defined as in claim 32; or
(b) acylating a compound of the formula IX, as defined above, with a compound of the formula 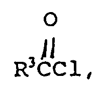 wherein R³ is defined as in claim 1, and then reducing the resulting amide.

3. A process for preparing a compound of the formula 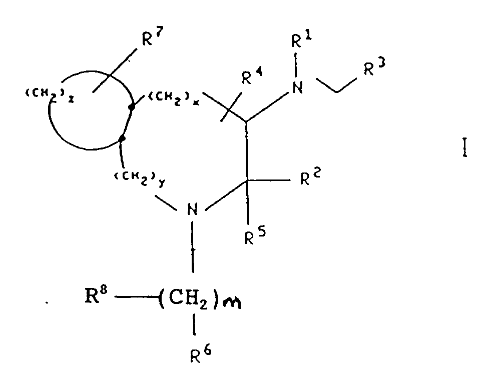 wherein x is an integer from zero to four;
y is an integer from zero to four;
z is an integer from one to six;
the ring containing (CH₂)_{z} may contain from zero to three double bonds, and one of the carbons of (CH₂)_{z} may optionally be replaced by oxygen, sulfur or nitrogen; m is an integer from zero to twelve, and any one of the carbon-carbon single bonds of (CH₂)ₘ may optionally be replaced by a carbon-carbon double or triple bond and any one of the carbon atoms of said (CH₂)ₘ may optionally be substituted with R⁸;
R¹ is hydrogen or (C₁-C₆)alkyl optionally substituted with hydroxy, alkoxy or fluoro;
R² is a radical selected from (C₅-C₇) cycloalkyl wherein one of the carbon atoms may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl groups may optionally be substituted with one or more substituents independently selected from halo, nitro, (C₁-C₆)alkyl, (C₁-C₆) alkoxy, trifluoromethyl, amino, (C₁-C₆)-alkylamino, 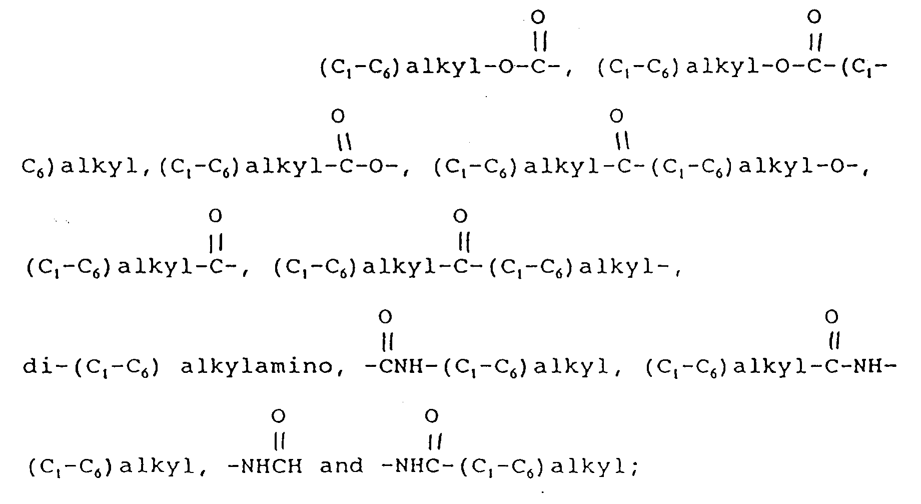 R⁵ is hydrogen or (C₁-C₆)alkyl; or R² and R⁵, together with the carbon to which they are attached, form a saturated carbocyclic ring having from 3 to 7 carbon atoms, wherein one of said carbon atoms may optionally be replaced by oxygen, nitrogen or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; or cycloalkyl having from three to seven carbon atoms, wherein one of said carbon atoms may optionally be replaced by nitrogen, oxygen or sulfur; wherein each of said aryl and heteroaryl groups may optionally be substituted with one or more substituents, and said (C₃-C₇) cycloalkyl may optionally be substituted with one or two substituents, said substitutents being independently selected from halo, nitro, (C₁-C₆) alkyl, (C₁-C₆) alkoxy, trifluoromethyl, phenyl, amino, (C₁-C₆)-alkylamino, 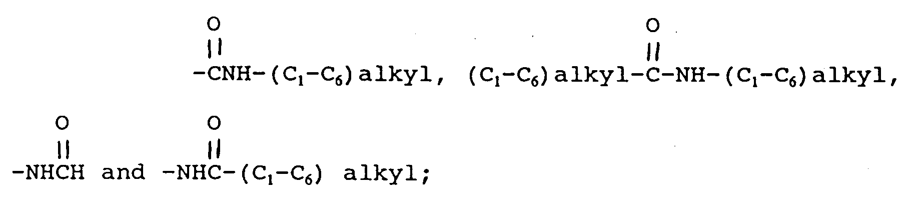 R⁴ may be attached to any atom of the nitrogen containing ring having an available bonding site and R⁷ may be attached to any atom of the (CH₂)_{z} containing ring having an available bonding site;
R⁴, R⁶, R⁷ and R⁸ are each independently selected from hydrogen, hydroxy, halo, amino, carboxy, carboxyalkyl, (C₁-C₆)alkylamino, di-(C₁-C₆)alkylamino, (C₁-C₆)alkoxy, 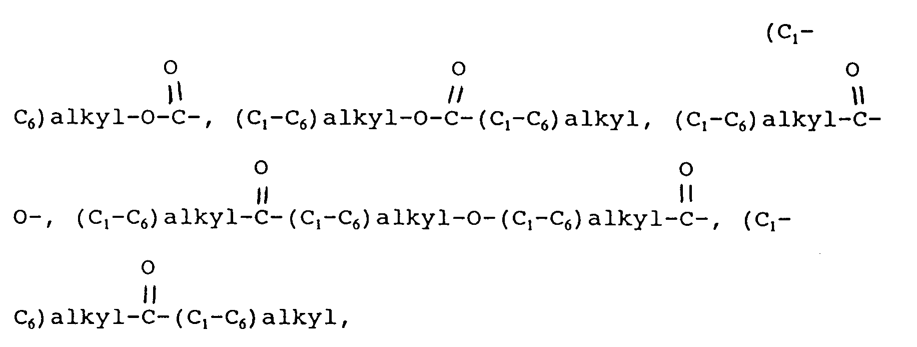 and the radicals set forth in the definition of R²,
or a pharmaceutically acceptable salt thereof,
comprising reacting a compound of the formula 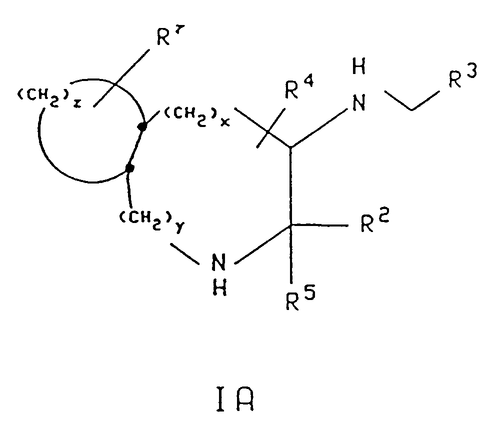 wherein R², R³, R⁴, R⁵, R⁷, x, y and z are defined as above, with a compound of the formula R⁶-(CH₂)ₘ-X, wherein m and R⁶ is defined as above and X is a leaving group, and wherein one of the carbon-carbon single bonds of (CH₂)ₘ may optionally be replaced by a carbon-carbon double bond and wherein one of the carbons of (CH₂)ₘ may optionally be substituted with R⁸, wherein R⁸ is defined as above.

4. A process for preparing a compound of the formula 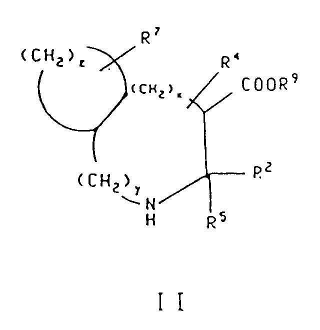 wherein R⁹ is (C₁-C₄)alkyl and R², R⁴, R⁵, R⁷, x, y and z are defined as in claim 3, comprising heating a compound of the formula 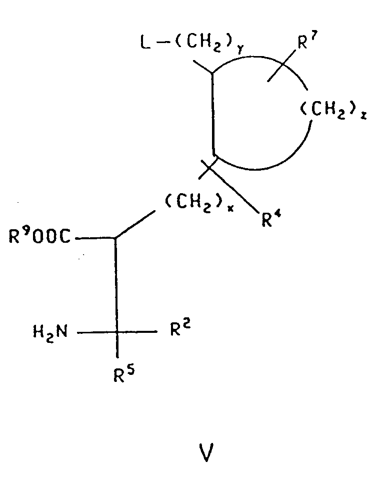 wherein R⁹ is (C₁-C₄) alkyl, L is a leaving group and R², R⁴, R⁵, R⁷, x, y and z are defined as in claim 3

5. A process according to claim 4, wherein said compound of formula V is obtained by reacting a β-lactam of the formula 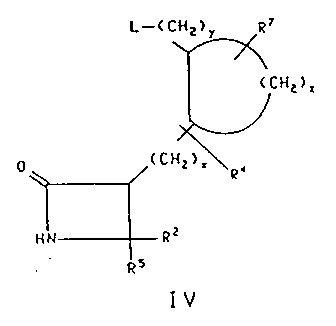 wherein L is a leaving group and R², R⁴, R⁵, R⁷, X, y and z are defined as in claim 3, with an acid.

6. A process according to claim 1 that produces a compound of the formula IA wherein y is zero, z is greater than one and R⁵ is hydrogen.

7. A process according to claim 2 that produces a compound of the formula IA, as defined in claim 1, wherein y is zero, z is greater than one and R⁵ is hydrogen.

8. A process for preparing a compound of the formula 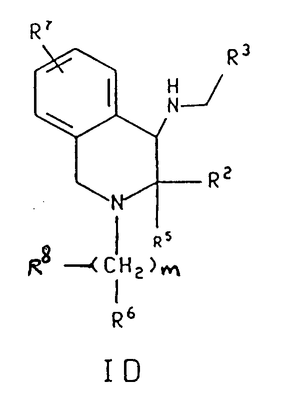 wherein R², R³, R⁵, R⁶, R⁷, R⁸ and m are defined as in claim 3, comprising reducing a compound of the formula 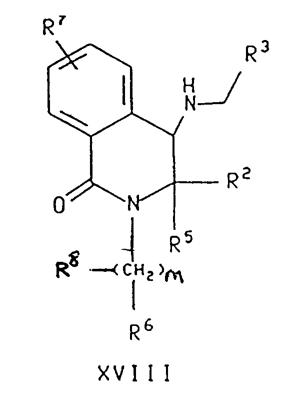 wherein R², R³, R⁵, R⁶, R⁷, R⁸ and m are defined as in claim 3.

9. A process according to claim 8; wherein said compound of the formula XVIII is obtained by either:
(a) reductive amination of a compound of the formula 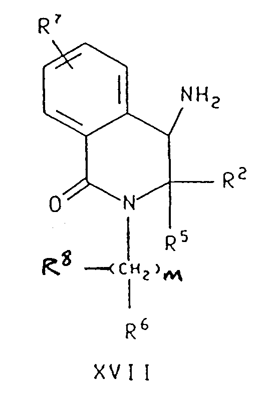 wherein R², R⁵, R⁶, R⁷, R⁸, and m are defined as in claim 3, or
(b) acylating a compound of the formula XVII, as defined above, with a compound of the formula 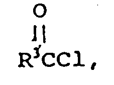 wherein R³ is defined as in claim 3, and then reducing the resulting amide.

## Patentansprüche (Patentansprüche für folgende(n) Vertragsstaat(en): AT, BE, CH, DE, DK, FR, GB, IT, LI, LU, NL, SE)

1. Verbindung der Formel 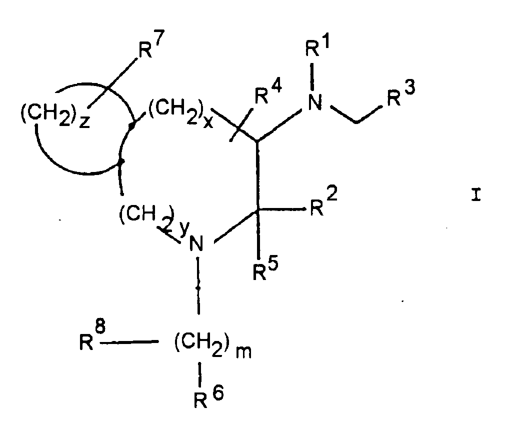 worin X eine ganze Zahl von null bis vier darstellt;
y eine ganze Zahl von null bis vier darstellt;
z eine ganze Zahl von eins bis sechs darstellt;
der Ring, der (CH₂)_{z} enthält, null bis drei Doppelbindungen enthalten kann und eines der Kohlenstoffatome von (CH₂)_{z} gegebenenfalls durch Sauerstoff, Schwefel oder Stickstoff ersetzt werden kann;
m eine ganze Zahl von null bis zwölf ist und eine der Kohlenstoff-Kohlenstoff-Einfachbindungen von (CH₂)ₘ gegebenenfalls durch eine Kohlenstoff-Kohlenstoff-Doppel- oder -Dreifachbindung ersetzt werden kann und eines der Kohlenstoffatome von (CH₂)ₘ gegebenenfalls mit R⁸ substituiert sein kann,
R¹ Wasserstoff oder (C₁-C₆)-Alkyl, gegebenenfalls substituiert mit Hydroxy, Alkoxy oder Fluor, ist;
R² ein Rest ist, ausgewählt aus
Aryl, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; Phenyl-(C₂-C₆)-alkyl, Benzhydryl und Benzyl, wobei jede der Aryl- und Heteroarylgruppen und die Phenylreste der Benzyl-, Phenyl-(C₂-C₆)-alkyl- und Benzhydrylgruppen gegebenenfalls substituiert mit einem oder mehreren Substituenten sein können, unabhängig ausgewählt aus Halogen, Nitro, (C₁-C₆)-Alkyl, (C₁-C₆)-Alkoxy, Trifluormethyl, Amino, (C₁-C₆)-Alkylamino, 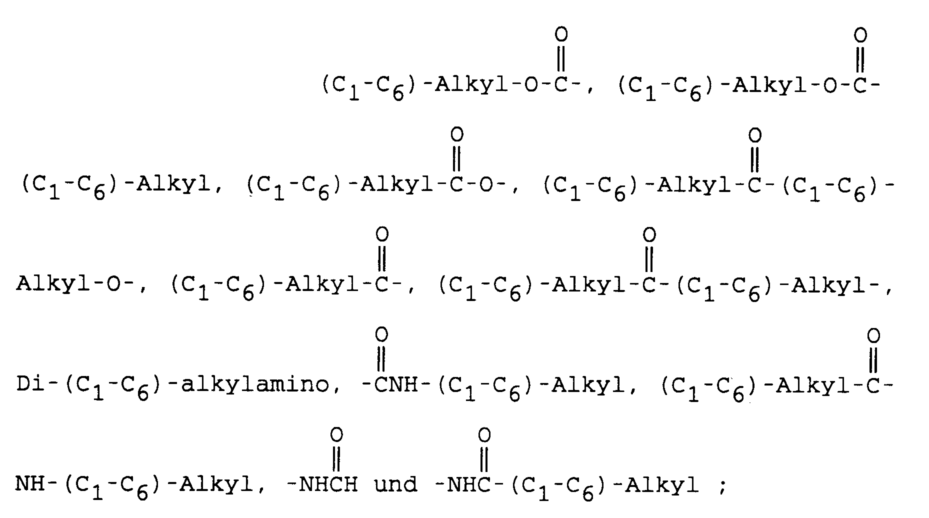 R⁵ Wasserstoff oder (C₁-C₆)-Alkyl ist;
oder R² und R⁵, zusammen mit dem Kohlenstoffatom, an das sie gebunden sind, einen gesättigten carbocyclischen Ring mit 3 bis 7 Kohlenstoffatomen bilden, wobei eines der Kohlenstoffatome gegebenenfalls durch Sauerstoff, Stickstoff oder Schwefel ersetzt werden kann;
R³ Aryl ist, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; oder Cycloalkyl mit drei bis sieben Kohlenstoffatomen, wobei eines der Kohlenstoffatome gegebenenfalls durch Stickstoff, Sauerstoff oder Schwefel ersetzt werden kann; wobei jede der Aryl- und Heteroarylgruppen gegebenenfalls mit einem oder mehreren Substituenten substituiert sein kann, und (C₃-C₇)-Cycloalkyl gegebenenfalls mit einem oder zwei Substituenten substituiert sein kann, wobei die Substituenten unabhängig ausgewählt sind aus Halogen, Nitro, (C₁-C₆)-Alkyl, (C₁-C₆)-Alkoxy, Trifluormethyl, Amino, Phenyl, (C₁-C₆)-Alkylamino, 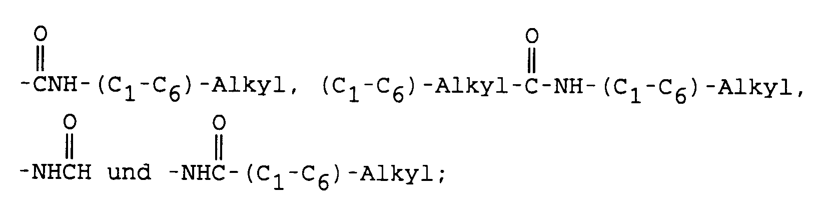 R⁴ an ein Atom des Stickstoff enthaltenden, eine verfügbare Bindungsstelle aufweisenden Ringes gebunden sein kann und R⁷ an ein Atom des (CH₂)_{z} enthaltenden, eine verfügbare Bindungsstelle aufweisenden Ringes gebunden sein kann;
R⁴, R⁶, R⁷ und R⁸ jeweils unabhängig voneinander ausgewählt sind aus Wasserstoff, Hydroxy, Halogen, Amino, Carboxy, Carboxyalkyl, (C₁-C₆)-Alkylamino, Di-(C₁-C₆)-alkylamino, (C₁-C₆)-Alkoxy, 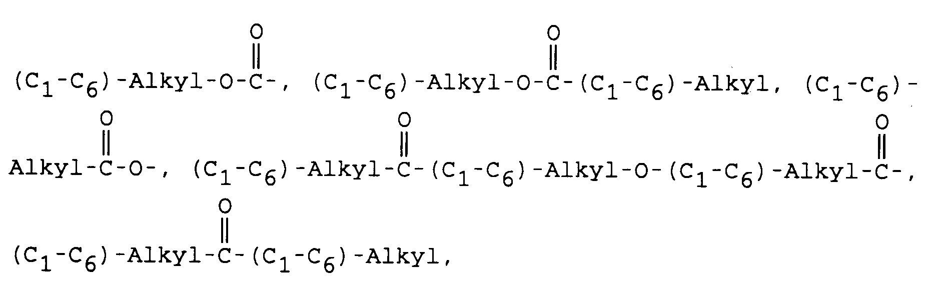 und den Resten gemäß Definition von R² oder ein pharmazeutisch verträgliches Salz davon.

2. Verbindung nach Anspruch 1, worin R¹, R⁴, R⁵, R⁶, R⁷ und R⁸ Wasserstoff sind; R² Phenyl darstellt; R³ Methoxyphenyl bedeutet, wobei der Phenylrest mit Wasserstoff, Chlor, Fluor, (C₁-C₆)-Alkoxy oder Trifluormethan substituiert ist; m null ist; und jeder Wert von x und y eine ganze Zahl von null bis vier ist und z eine ganze Zahl von eins bis vier ist.

3. Verbindung nach Anspruch 1, wobei die Verbindung [1α,3α,4α,5α]-4-(2-Methoxybenzyl)amino-3-phenyl-2-azabicyclo[3.3.0]octan ist.

4. Verbindung nach Anspruch 1, wobei die Verbindung 4-(2-Methoxybenzyl)amino-3-phenyl-2-azabicyclo[4.4.0]decan ist.

5. Verbindung nach Anspruch 1, wobei die Verbindung 5-(2-Methoxybenzyl)amino-4-benzhydryl-3-azabicyclo[4.1.0]heptan ist.

6. Verbindung nach Anspruch 1, wobei die Verbindung 3-(2-Methoxybenzyl)amino-2-phenyl-1,2,3,4-tetrahydrochinolin ist.

7. Verbindung nach Anspruch 1, wobei die Verbindung trans-4-(2-Methoxybenzylamino)-2-methyl-3-phenyl-3,4-dihydro-1(2H)-isochinolinon ist.

8. Verbindung nach Anspruch 1, wobei die Verbindung trans-4-(2-Methoxybenzylamino)-3-phenyl-3,4-dihydro-1(2H)-isochinolinon ist.

9. Verbindung nach Anspruch 1, wobei die Verbindung trans-4-(2-Methoxybenzylamino)-2-methyl-3-phenyl-1,2,3,4-tetrahydroisochinolin ist.

10. Verbindung nach Anspruch 1, wobei die Verbindung trans-4-(2-Methoxybenzylamino)-3-phenyl-1,2,3,4-tetrahydroisochinolindihydrochlorid ist.

11. Verbindung nach Anspruch 1, wobei die Verbindung 5-(2-Methoxybenzyl)amino-4-benzhydryl-3-azabicyclo[4.4.0]decan ist.

12. Verbindung nach Anspruch 1, wobei die Verbindung [1α,3α,4β,5α]-4-(2-Methoxybenzyl)amino-3-phenyl-2-azabicyclo[3.3.0]octan ist.

13. Verbindung nach Anspruch 1, wobei die Verbindung [1α,3α,4β,5α]-4-(2-Chlorbenzyl)amino-3-phenyl-2-azabicyclo[3.3.0]octan ist.

14. Verbindung nach Anspruch 1, wobei die Verbindung [1α,3α,4α,5α]-4-(2,5-Dimethoxybenzyl)amino-3-phenyl-2-azabicyclo[3.3.0]octan ist.

15. Verbindung nach Anspruch 1, wobei die Verbindung [1α,3α,4α,5α]-4-(2-Methoxy-5-chlorbenzyl)amino-3-phenyl-2-azabicyclo[3.3.0]octan ist.

16. Verbindung nach Anspruch 1, worin die Verbindung [1α,3α,4α,5α]-4-(2-Methoxy-5-methylbenzyl)amino-3-phenyl-2-azabicyclo[3.3.0]octan ist.

17. Verbindung der Formel 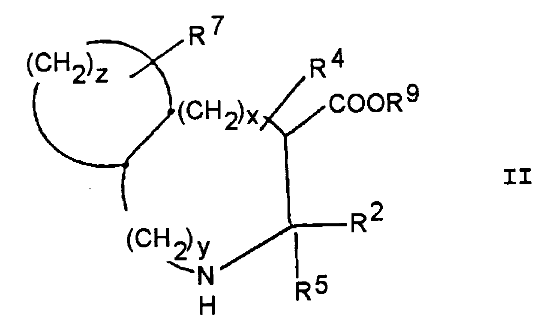 worin R², R⁴, R⁵, R⁷, x, y und z wie in Anspruch 1 definiert sind und R⁹ (C₁-C₆)-Alkyl oder Wasserstoff ist.

18. Pharmazeutische Zusammensetzung, umfassend eine Verbindung nach Anspruch 1 und einen pharmazeutisch verträglichen Träger.

## Patentansprüche (Patentansprüche für folgende(n) Vertragsstaat(en): ES, GR)

1. Verfahren zur Herstellung einer Verbindung der Formel 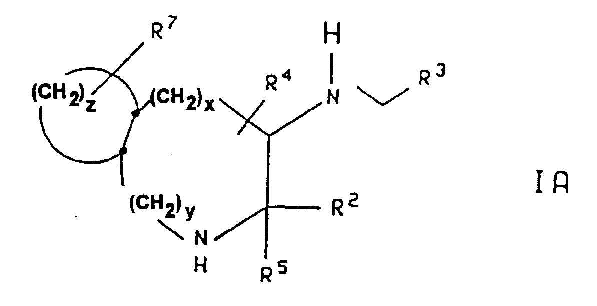 worin x eine ganze Zahl von null bis vier darstellt;
y eine ganze Zahl von null bis vier darstellt;
z eine ganze Zahl von eins bis sechs darstellt;
der Ring, der (CH₂)_{z} enthält, null bis drei Doppelbindungen enthalten kann und eines der Kohlenstoffatome von (CH₂)_{z} gegebenenfalls durch Sauerstoff, Schwefel oder Stickstoff ersetzt werden kann;
R² ein Rest ist, ausgewählt aus
Aryl, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; Phenyl-(C₂-C₆)-alkyl, Benzhydryl und Benzyl, wobei jede der Aryl- und Heteroarylgruppen und die Phenylreste der Benzyl-, Phenyl-(C₂-C₆)-alkyl- und Benzhydrylgruppen gegebenenfalls substituiert mit einem oder mehreren Substituenten sein können, unabhängig ausgewählt aus Halogen, Nitro, (C₁-C₆)-Alkyl, (C₁-C₆)-Alkoxy, Trifluormethyl, Amino, (C₁-C₆)-Alkylamino, 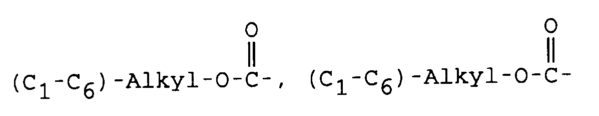 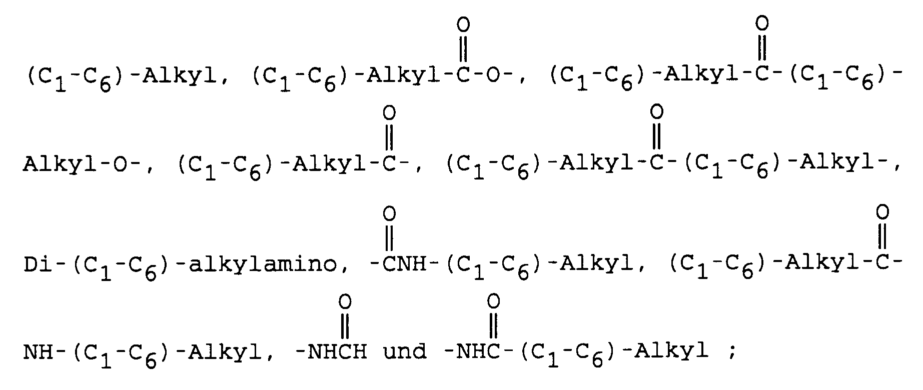 R⁵ Wasserstoff oder (C₁-C₆)-Alkyl ist;
oder R² und R⁵, zusammen mit dem Kohlenstoffatom, an das sie gebunden sind, einen gesättigten carbocyclischen Ring mit 3 bis 7 Kohlenstoffatomen bilden, wobei eines der Kohlenstoffatome gegebenenfalls durch Sauerstoff, Stickstoff oder Schwefel ersetzt werden kann;
R³ Aryl, ausgewählt ist aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; oder Cycloalkyl mit drei bis sieben Kohlenstoffatomen, wobei eines der Kohlenstoffatome gegebenenfalls durch Stickstoff, Sauerstoff oder Schwefel ersetzt werden kann; wobei jede der Aryl- und Heteroarylgruppen gegebenenfalls mit einem oder mehreren Substituenten substituiert sein kann, und (C₃-C₇)-Cycloalkyl, gegebenenfalls mit einem oder zwei Substituenten substituiert sein kann, wobei die Substituenten unabhängig ausgewählt sind aus Halogen, Nitro, (C₁-C₆)-Alkyl, (C₁-C₆)-Alkoxy, Trifluormethyl, Amino, Phenyl, (C₁-C₆)-Alkylamino, 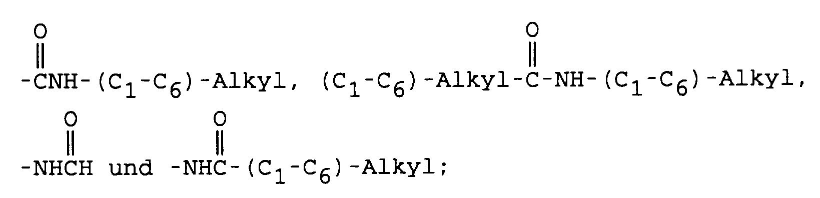 R⁴ an ein Atom des Stickstoff enthaltenden, eine verfügbare Bindungsstelle aufweisenden Ringes gebunden sein kann und R⁷ an ein Atom des (CH₂)_{z} enthaltenden, eine verfügbare Bindungsstelle aufweisenden Ringes gebunden sein kann;
R⁴ und R⁷ jeweils unabhängig voneinander ausgewählt sind aus Wasserstoff, Hydroxy, Halogen, Amino, Carboxy, Carboxyalkyl, (C₁-C₆)-Alkylamino, Di-(C₁-C₆)-alkylamino, (C₁-C₆)-Alkoxy, 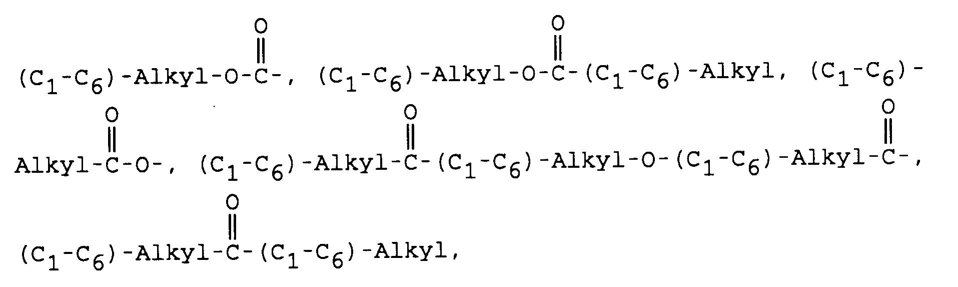 und den Resten gemäß Definition von R² oder ein pharmazeutisch verträgliches Salz davon,
umfassend Umsetzen einer Verbindung der Formel 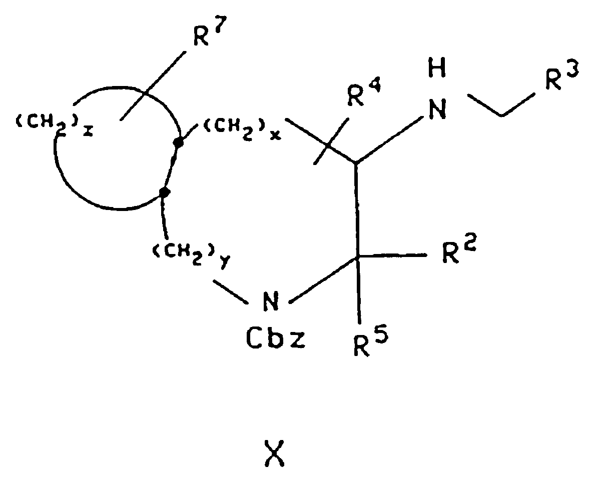 worin R², R³, R⁴, R⁵, R⁷, x, y und z wie vorstehend definiert sind und cbz Carbobenzyloxy ist, mit Ammoniumformiat in Gegenwart von Palladium-auf-Kohle.

2. Verfahren nach Anspruch 1, worin die Verbindung der Formel X erhalten wird durch entweder:
(a) reduktive Aminierung einer Verbindung der Formel
R³CHO, worin R³ wie in Anspruch 1 definiert ist, in Gegenwart einer Verbindung der Formel 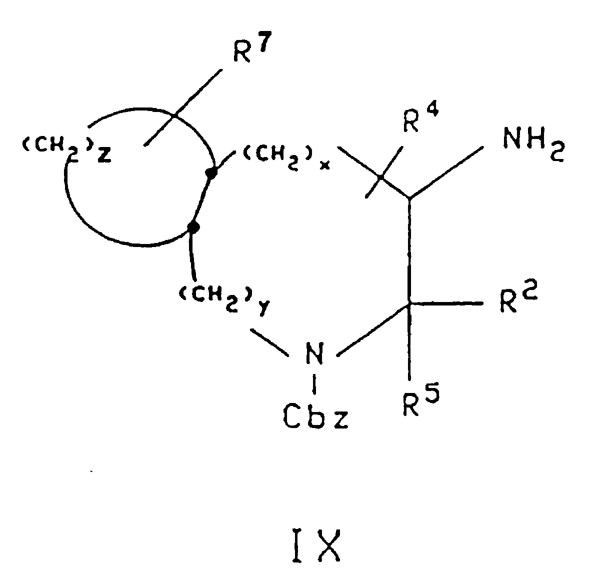 worin R², R³, R⁴, R⁵, R⁷, x, y und z wie in Anspruch definiert 32 sind; oder
(b) Acylieren einer Verbindung der Formel IX, wie vorstehend definiert, mit einer Verbindung der Formel 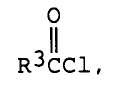 worin R³ wie in Anspruch 1 definiert ist, und anschließend Reduzieren des erhaltenen Amids.

3. Verfahren zur Herstellung einer Verbindung der Formel 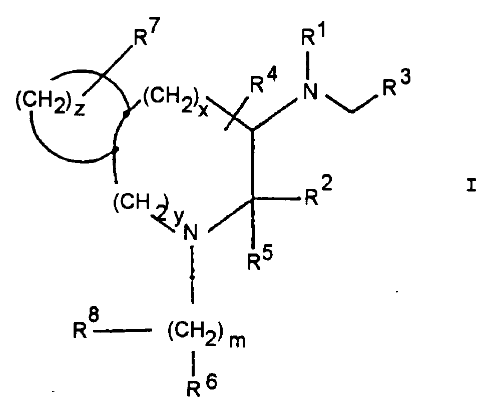 worin x eine ganze Zahl von null bis vier darstellt;
y eine ganze Zahl von null bis vier darstellt;
z eine ganze Zahl von eins bis sechs darstellt;
der Ring, der (CH₂)_{z} enthält, null bis drei Doppelbindungen enthalten kann und eines der Kohlenstoffatome von (CH₂)_{z} gegebenenfalls durch Sauerstoff, Schwefel oder Stickstoff ersetzt werden kann;
m eine ganze Zahl von null bis zwölf ist und eine der Kohlenstoff-Kohlenstoff-Einfachbindungen von (CH₂)ₘ gegebenenfalls durch eine Kohlenstoff-Kohlenstoff-Doppel- oder -Dreifachbindung ersetzt werden kann und eines der Kohlenstoffatome von (CH₂)ₘ gegebenenfalls mit R⁸ substituiert sein kann;
R¹ Wasserstoff oder (C₁-C₆)-Alkyl ist, gegebenenfalls substituiert mit Hydroxy, Alkoxy oder Fluor;
R² ein Rest ist, ausgewählt aus
(C₅-C₇)-Cycloalkyl, worin eines der Kohlenstoffatome gegebenenfalls durch Stickstoff, Sauerstoff oder Schwefel ersetzt sein kann; Aryl, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; Phenyl-(C₂-C₆)-alkyl, Benzhydryl und Benzyl, wobei jede der Aryl- und Heteroarylgruppen und die Phenylreste der Benzyl-, Phenyl-(C₂-C₆)-alkyl- und Benzhydrylgruppen gegebenenfalls substituiert sein können mit einem oder mehreren Substituenten, unabhängig ausgewählt aus Halogen, Nitro, (C₁-C₆)-Alkyl, (C₁-C₆)-Alkoxy, Trifluormethyl, Amino, (C₁-C₆)-Alkylamino, 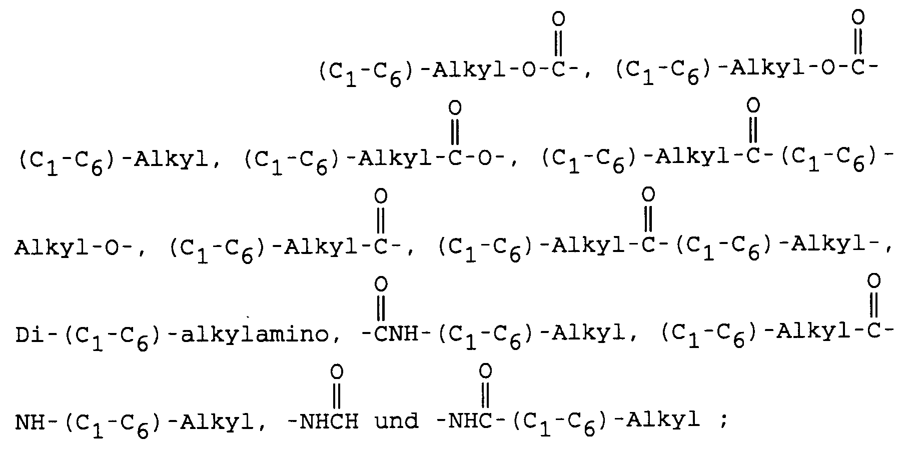 R⁵ Wasserstoff oder (C₁-C₆)-Alkyl ist;
oder R² und R⁵, zusammen mit dem Kohlenstoffatom, an das sie gebunden sind, einen gesättigten carbocyclischen Ring mit 3 bis 7 Kohlenstoffatomen bilden, worin eines der Kohlenstoffatome gegebenenfalls durch Sauerstoff, Stickstoff oder Schwefel ersetzt werden kann;
R³ Aryl ist, ausgewählt aus Phenyl und Naphthyl; Heteroaryl, ausgewählt aus Indanyl, Thienyl, Furyl, Pyridyl, Thiazolyl, Isothiazolyl, Oxazolyl, Isoxazolyl, Triazolyl, Tetrazolyl und Chinolyl; oder Cycloalkyl mit drei bis sieben Kohlenstoffatomen, worin eines der Kohlenstoffatome gegebenenfalls durch Stickstoff, Sauerstoff oder Schwefel ersetzt werden kann; wobei jede der Aryl- und Heteroarylgruppen gegebenenfalls mit einem oder mehreren Substituenten substituiert sein kann, und (C₃-C₇)-Cycloalkyl gegebenenfalls mit einem oder zwei Substituenten substituiert sein kann, wobei die Substituenten unabhängig ausgewählt sind aus Halogen, Nitro, (C₁-C₆)-Alkyl, (C₁-C₆)-Alkoxy, Trifluormethyl, Phenyl, Amino, (C₁-C₆)-Alkylamino, 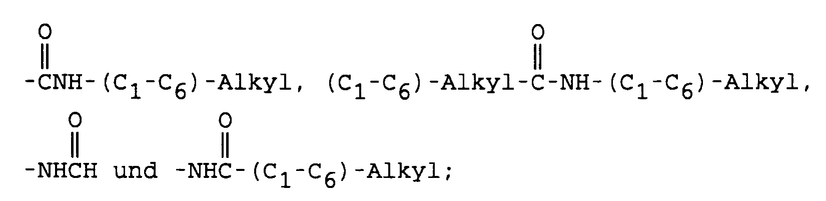 R⁴ an ein Atom des Stickstoff enthaltenden, eine verfügbare Bindungsstelle aufweisenden Ringes gebunden sein kann und R⁷ an ein Atom des (CH₂)_{z} enthaltenden, eine verfügbare Bindungsstelle aufweisenden Ringes gebunden sein kann;
R⁴, R⁶, R⁷ und R⁸ jeweils unabhängig voneinander ausgewählt sind aus Wasserstoff, Hydroxy, Halogen, Amino, Carboxy, Carboxyalkyl, (C₁-C₆)-Alkylamino, Di-(C₁-C₆)-alkylamino, (C₁-C₆)-Alkoxy, 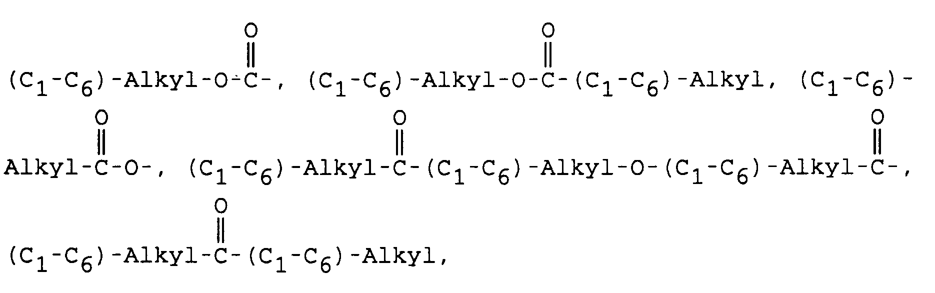 und den Resten gemäß Definition von R² oder ein pharmazeutisch verträgliches Salz davon,
umfassend Umsetzen einer Verbindung der Formel 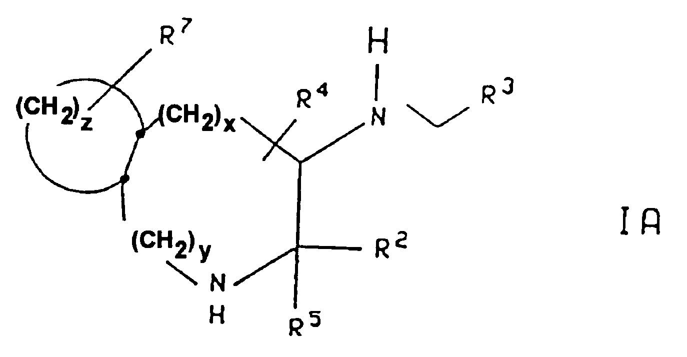 worin R², R³, R⁴, R⁵, R⁷, x, y und z wie vorstehend definiert sind, mit einer Verbindung der Formel R⁶-(CH₂)ₘ-X, worin m und R⁶ wie vorstehend definiert sind und X eine Abgangsgruppe darstellt und worin eine der Kohlenstoff-Kohlenstoff-Einfachbindungen von (CH₂)ₘ gegebenenfalls durch eine Kohlenstoff-Kohlenstoff-Doppelbindung ersetzt werden kann und worin eines der Kohlenstoffatome von (CH₂)ₘ gegebenenfalls mit R⁸, wobei R⁸ wie vorstehend definiert ist, substituiert sein kann.

4. Verfahren zur Herstellung einer Verbindung der Formel 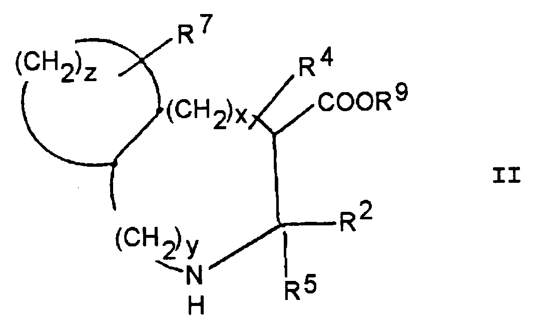 worin R⁹ (C₁-C₄)-Alkyl darstellt und R², R⁴, R⁵, R⁷, x, y und z wie in Anspruch 3 definiert sind, umfassend Erhitzen einer Verbindung der Formel 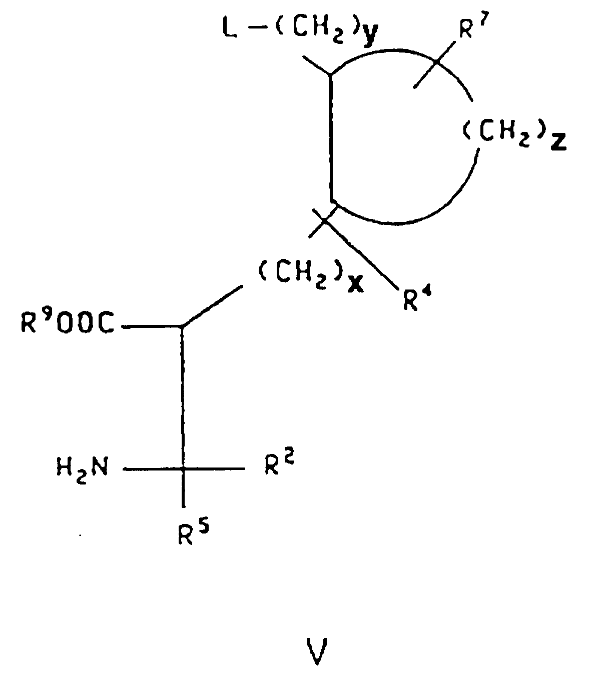 worin R⁹ (C₁-C₄)-Alkyl ist, L eine Abgangsgruppe darstellt und R², R⁴, R⁵, R⁷, x, y und z wie in Anspruch 3 definiert sind.

5. Verfahren nach Anspruch 4, wobei die Verbindung der Formel V durch Umsetzen eines β-Lactams der Formel 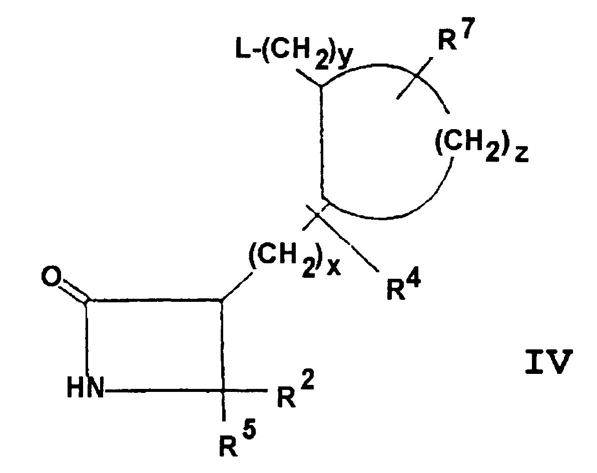 worin L eine Abgangsgruppe darstellt und R², R⁴, R⁵, R⁷, x, y und z wie in Anspruch 3 definiert sind, mit einer Säure erhalten wird.

6. Verfahren nach Anspruch 1, zur Herstellung einer Verbindung der Formel IA, worin y null ist, z größer als eins ist und R⁵ Wasserstoff ist.

7. Verfahren nach Anspruch 2, zur Herstellung einer Verbindung der Formel IA, gemäß Anspruch 1, worin y null ist, z größer als eins ist und R⁵ Wasserstoff ist.

8. Verfahren zur Herstellung einer Verbindung der Formel 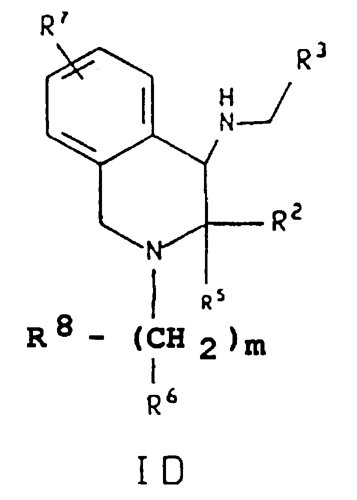 worin R², R³, R⁵, R⁶, R⁷, R⁸ und m wie in Anspruch 3 definiert sind, umfassend Reduktion einer Verbindung der Formel 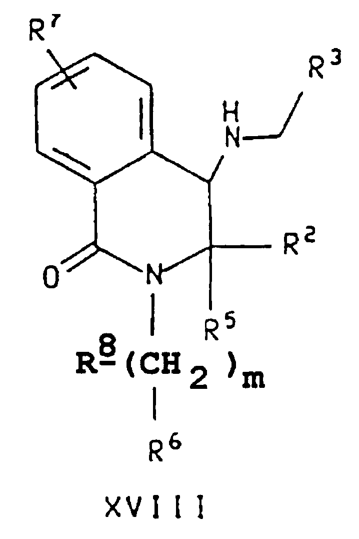 worin R², R³, R⁵, R⁶, R⁷, R⁸ und m wie in Anspruch 3 definiert sind.

9. Verfahren nach Anspruch 8, wobei die Verbindung der Formel XVIII erhalten wird durch entweder:
(a) reduktive Aminierung einer Verbindung der Formel 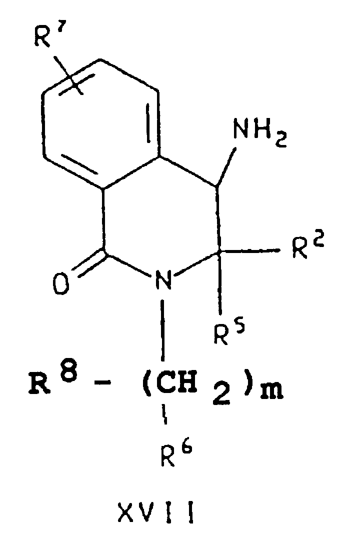 worin R², R⁵; R⁶, R⁷, R⁸ und m wie in Anspruch definiert 3 sind; oder
(b) Acylieren einer Verbindung der Formel XVII, wie vorstehend definiert, mit einer Verbindung der Formel 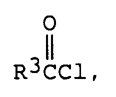 worin R³ wie in Anspruch 3 definiert ist, und anschließend Reduzieren des erhaltenen Amids.

## Revendications (Revendications pour l'(les) Etat(s) contractant(s) suivant(s): AT, BE, CH, DE, DK, FR, GB, IT, LI, LU, NL, SE)

1. Composé de formule 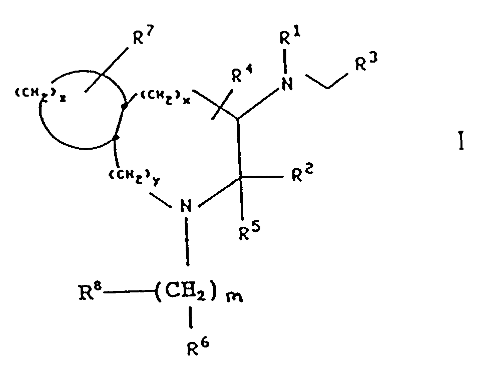 dans laquelle x représente un nombre entier de zéro à quatre ;
y représente un nombre entier de zéro à quatre ;
z représente un nombre entier de un à six ;
le noyau contenant le groupe (CH₂)_{z} peut contenir zéro à trois doubles liaisons, et un des atomes de carbone du groupe (CH₂)_{z} peut être remplacé facultativement par l'oxygène, le soufre ou l'azote ;
m est un nombre entier de zéro à douze, et l'une quelconque des simples liaisons carbone-carbone du groupe (CH₂)ₘ peut être facultativement remplacée par une double ou triple liaison carbone-carbone et l'un quelconque des atomes de carbone dudit groupe (CH₂)ₘ peut être facultativement substitué avec un groupe R⁸ ;
R¹ représente l'hydrogène ou un groupe alkyle en C₁ à C₆ facultativement substitué avec un groupe hydroxy, alkoxy ou fluoro ;
R² représente un radical choisi entre
un radical aryle choisi entre les radicaux phényle et naphtyle ; un radical hétéro-aryle choisi entre les radicaux indanyle, thiényle, furyle, pyridyle, thiazolyle, isothiazolyle, oxazolyle, isoxazolyle, triazolyle, tétrazolyle et quinolyle ; des radicaux phényl-(alkyle en C₂ à C₆), benzhydryle et benzyle, dans lesquels chacun desdits groupes aryle et hétéro-aryle et les groupements phényle desdits groupes benzyle, phényl-(alkyle en C₂ à C₆) et benzhydryle peut porter facultativement un ou plusieurs substituants choisis, indépendamment, entre des substituants halogéno, nitro, alkyle en C₁ à C₆, alkoxy en C₁ à C₆, trifluorométhyle, amino, alkylamino en C₁ à C₆, 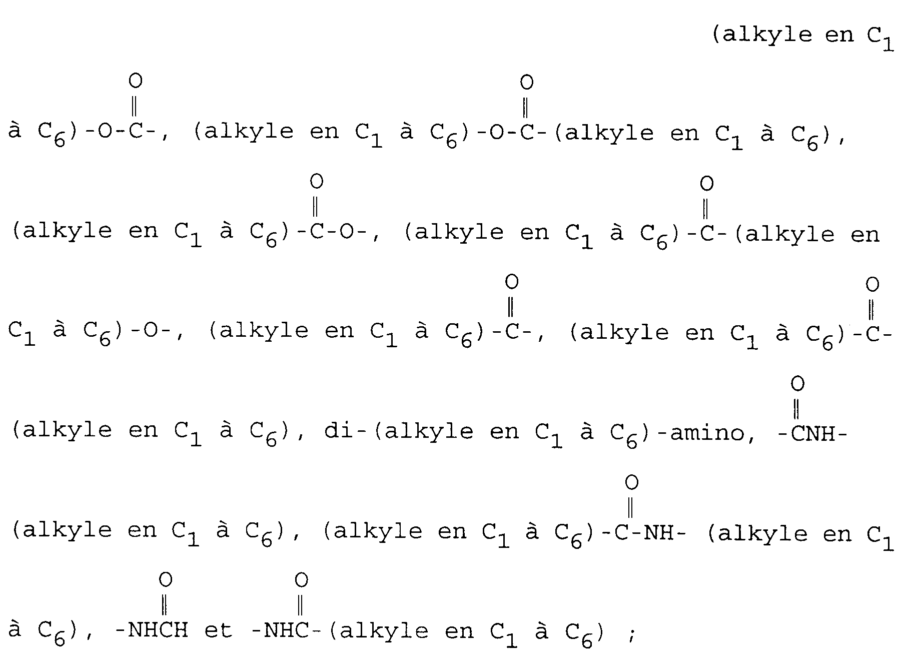 R⁵ représente l'hydrogène ou un groupe alkyle en C₁ à C₆ ;
ou bien R² et R⁵, conjointement avec l'atome de carbone auquel ils sont fixés, forment un noyau carbocyclique saturé ayant 3 à 7 atomes de carbone, dans lequel un de ces atomes de carbone peut être facultativement remplacé par l'oxygène, l'azote ou le soufre ;
R³ représente un radical aryle choisi entre les radicaux phényle et naphtyle ; un radical hétéro-aryle choisi entre les radicaux indanyle, thiényle, furyle, pyridyle, thiazolyle, isothiazolyle, oxazolyle, isoxazolyle, triazolyle, tétrazolyle et quinolyle ; ou un radical cycloalkyle ayant trois à sept atomes de carbone, dans lequel un de ces atomes de carbone peut être facultativement remplacé par l'azote, l'oxygène ou le soufre ; chacun desdits groupes aryle et hétéro-aryle pouvant porter facultativement un ou plusieurs substituants, et ledit groupe cycloalkyle en C₃ à C₇ pouvant porter facultativement un ou deux substituants, lesdits substituants étant choisis, indépendamment, entre des substituants halogéno, nitro, alkyle en C₁ à C₆, alkoxy en C₁ à C₆, trifluorométhyle, amino, phényle, alkylamino en C₁ à C₆, 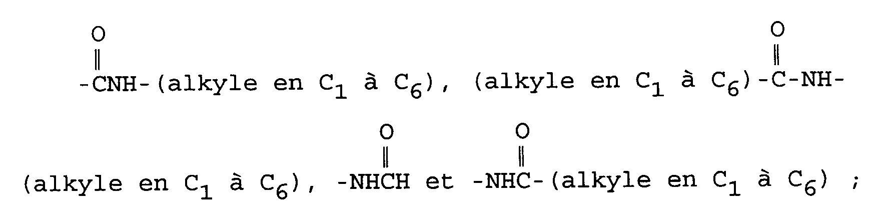 R⁴ peut être fixé à n'importe quel atome du noyau contenant de l'azote possédant un site de liaison disponible et R⁷ peut être fixé à n'importe quel atome du noyau contenant le groupe (CH₂)_{z}, ayant un site de liaison disponible ;
R⁴, R⁶, R⁷ et R⁸ sont choisis chacun, indépendamment, entre l'hydrogène, des groupes hydroxy, halogéno, amino, carboxy, carboxyalkyle, alkylamino en C₁ à C₆, di(alkyle en C₁ à C₆)-amino, alkoxy en C₁ à C₆, 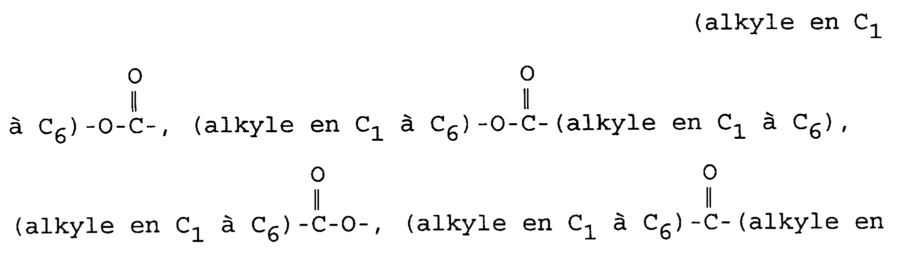 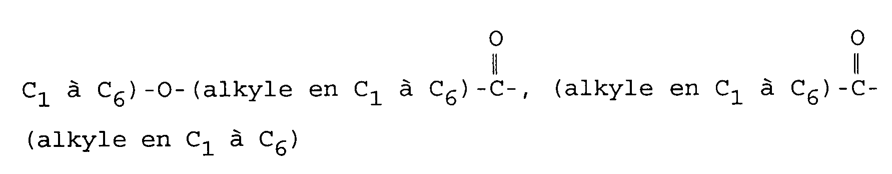 et les radicaux indiqués dans la définition de R² ;
ou un de ses sels pharmaceutiquement acceptables.

2. Composé suivant la revendication 1, dans lequel R¹, R⁴, R⁵, R⁶, R⁷ et R⁸ représentent l'hydrogène, R² représente un groupe phényle ; R³ représente un groupe méthoxyphényle dans lequel le groupement phényle est substitué avec l'hydrogène, le chlore, le fluor, un groupe alkoxy en C₁ à C₆ ou trifluorométhane ; m est égal à zéro ; et chacun des indices x et y est un nombre entier de zéro à quatre, et z est un nombre entier de un à quatre.

3. Composé suivant la revendication 1, qui est le [1α,3α,4α,5α]-4-(2-méthoxybenzyl)amino-3-phényl-2-azabicyclo[3.3.0]octane.

4. Composé suivant la revendication 1, qui est le 4-(2-méthoxybenzyl)amino-3-phényl-2-azabicyclo[4.4.0]décane.

5. Composé suivant la revendication 1, qui est le 5-(2-méthoxybenzyl)amino-4-benzhydryl-3-azabicyclo[4.1.0]heptane.

6. Composé suivant la revendication 1, qui est la 3-(2-méthoxybenzyl)amino-2-phényl-1,2,3,4-tétrahydroquinoléine.

7. Composé suivant la revendication 1, qui est la trans-4-(2-méthoxybenzylamino)-2-méthyl-3-phényl-3,4-dihydro-1(2H)-isoquinolinone.

8. Composé suivant la revendication 1, qui est la trans-4-(2-méthoxybenzylamino)-3-phényl-3,4-dihydro-1(2H)-isoquinolinone.

9. Composé suivant la revendication 1, qui est la trans-4-(2-méthoxybenzylamino)-2-méthyl-3-phényl-1,2,3,4-tétrahydroisoquinoléine.

10. Composé suivant la revendication 1, qui est le dichlorhydrate de trans-4-(2-méthoxybenzylamino)-3-phényl-1,2,3,4-tétrahydroisoquinoléine.

11. Composé suivant la revendication 1, qui est le 5-(2-méthoxybenzyl)amino-4-benzhydryl-3-azabicyclo[4.4.0]décane.

12. Composé suivant la revendication 1, qui est le [1α,3α,4β,5α]-4-(2-méthoxybenzyl)amino-3-phényl-2-azabicyclo[3.3.0]octane.

13. Composé suivant la revendication 1, qui est le [1α,3α,4β,5α]-4-(2-chlorobenzyl)amino-3-phényl-2-azabicyclo[3.3.0]octane.

14. Composé suivant la revendication 1, qui est le [1α,3α,4α,5α]-4-(2,5-diméthoxybenzyl)amino-3-phényl-2-azabicyclo[3.3.0]octane.

15. Composé suivant la revendication 1, qui est le [1α,3α,4α,5α]-4-(2-méthoxy-5-chlorobenzyl)amino-3-phényl-2-azabicyclo[3.3.0]octane.

16. Composé suivant la revendication 1, qui est le [1α,3α,4α,5α]-4-(2-méthoxy-5-méthylbenzyl)amino-3-phényl-2-azabicyclo[3.3.0]octane.

17. Composé de formule 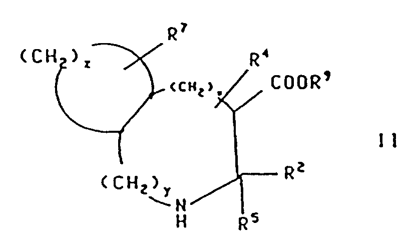 dans laquelle R², R⁴, R⁵, R⁷, x, y et z répondent aux définitions suivant la revendication 1, et R⁹ représente un groupe alkyle en C₁ à C₆ ou l'hydrogène.

18. Composition pharmaceutique comprenant un composé suivant la revendication 1 et un support pharmaceutiquement acceptable.

## Revendications (Revendications pour l'(les) Etat(s) contractant(s) suivant(s): ES, GR)

1. Procédé de préparation d'un composé de formule 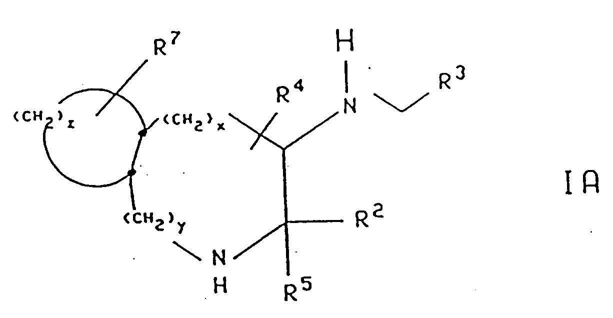 dans laquelle x est un nombre entier de zéro à quatre ;
y est un nombre entier de zéro à quatre ;
z est un nombre entier de un à six ;
le noyau contenant le groupe (CH₂)_{z} peut contenir zéro à trois doubles liaisons, et un des atomes de carbone du groupe (CH₂)_{z} peut être remplacé facultativement par l'oxygène, le soufre ou l'azote ;
R² est un radical choisi entre
un radical aryle choisi entre les radicaux phényle et naphtyle ; un radical hétéro-aryle choisi entre les radicaux indanyle, thiényle, furyle, pyridyle, thiazolyle, isothiazolyle, oxazolyle, isoxazolyle, triazolyle, tétrazolyle et quinolyle ; les radicaux phényl-(alkyle en C₂ à C₆), benzhydryle et benzyle, dans lesquels chacun desdits groupes aryle et hétéro-aryle et les groupements phényle desdits groupes benzyle, phényl-(alkyle en C₂ à C₆) et benzhydryle peut porter facultativement un ou plusieurs substituants choisis, indépendamment, entre des substituants halogéno, nitro, alkyle en C₁ à C₆, alkoxy en C₁ à C₆, trifluorométhyle, amino, alkylamino en C₁ à C₆, 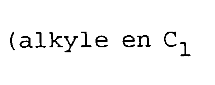 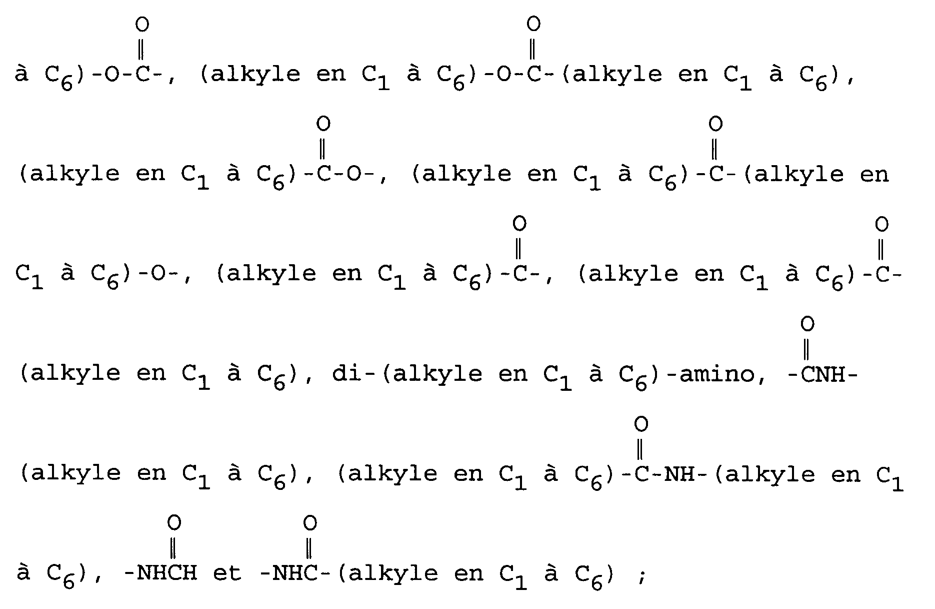 R⁵ représente l'hydrogène ou un groupe alkyle en C₁ à C₆ ;
ou bien R² et R⁵, conjointement avec l'atome de carbone auquel ils sont fixés, forment un noyau carbocyclique saturé ayant 3 à 7 atomes de carbone, dans lequel un desdits atomes de carbone peut être facultativement remplacé par l'oxygène, l'azote ou le soufre ;
R³ représente un radical aryle choisi entre les radicaux phényle et naphtyle ; un radical hétéro-aryle choisi entre les radicaux indanyle, thiényle, furyle, pyridyle, thiazolyle, isothiazolyle, oxazolyle, isoxazolyle, triazolyle, tétrazolyle et quinolyle ; ou un radical cycloalkyle ayant trois à sept atomes de carbone, dans lequel un desdits atomes de carbone peut être facultativement remplacé par l'azote, l'oxygène ou le soufre ; chacun desdits groupes aryle et hétéro-aryle pouvant porter facultativement un ou plusieurs substituants, et ledit groupe cycloalkyle en C₃ à C₇ pouvant porter facultativement un ou deux substituants, lesdits substituants étant choisis, indépendamment, entre des substituants halogéno, nitro, alkyle en C₁ à C₆, alkoxy en C₁ à C₆, trifluorométhyle, amino, phényle, alkylamino en C₁ à C₆, 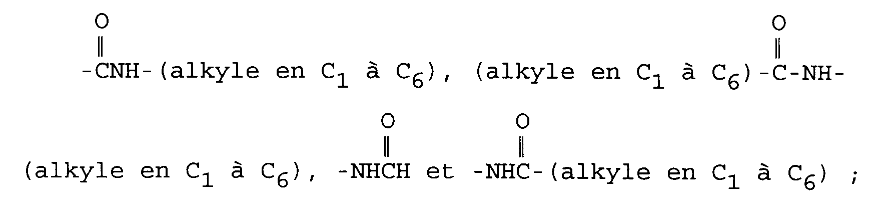 R⁴ peut être fixé à n'importe quel atome du noyau contenant de l'azote ayant un site de liaison disponible et R⁷ peut être fixé à n'importe quel atome du noyau contenant le groupe (CH₂)_{z}, ayant un site de liaison disponible ;
R⁴ et R⁷ sont choisis chacun, indépendamment, entre l'hydrogène, des groupes hydroxy, halogéno, amino, carboxy, carboxyalkyle, alkylamino en C₁ à C₆, di-(alkyle en C₁ à C₆)-amino, alkoxy en C₁ à C₆, 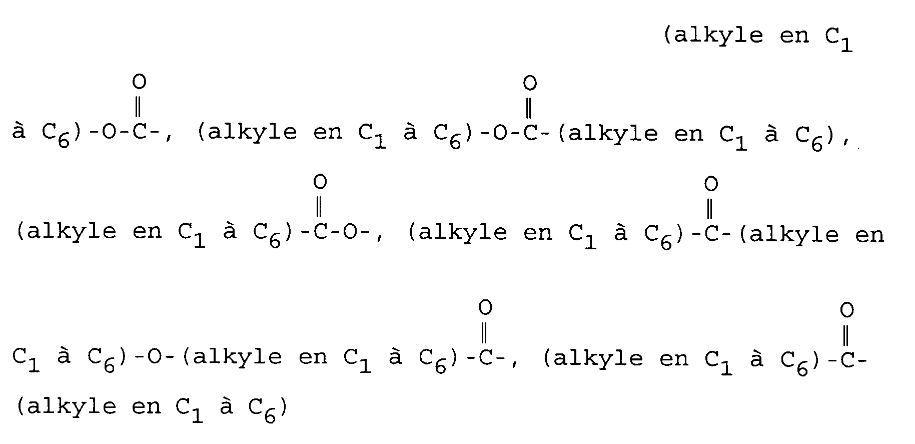 et les radicaux indiqués dans la définition de R² ;
ou un de ses sels pharmaceutiquement acceptables,
comprenant la réaction d'un composé de formule 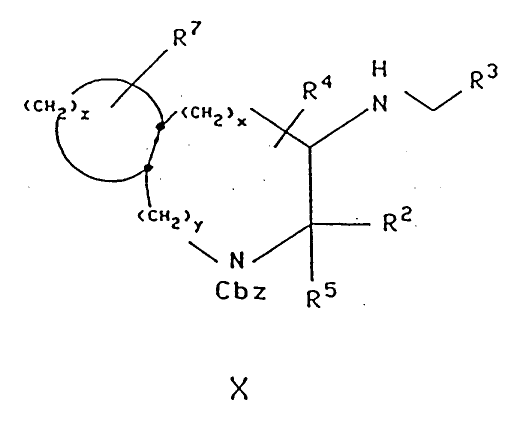 dans laquelle R², R³, R⁴, R⁵, R⁷, x, y et z répondent aux définitions précitées et cbz représente un groupe carbobenzyloxy, avec le formiate d'ammonium en présence de palladium sur du charbon.

2. Procédé suivant la revendication 1, dans lequel le composé de formule X est obtenu par :
(a) amination réductrice d'un composé de formule R³CHO, dans laquelle R³ répond à la définition suivant la revendication 1, en présence d'un composé de formule 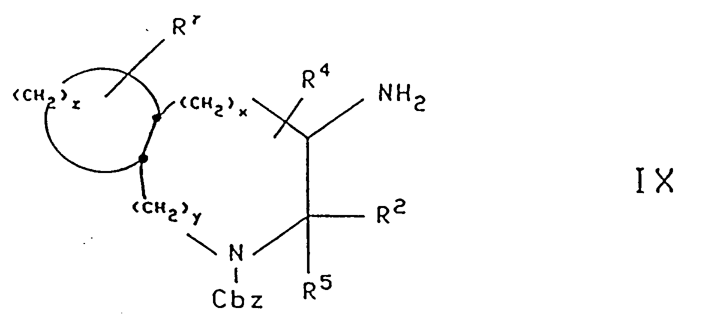 dans laquelle R², R³, R⁴, R⁵, R⁷, x, y et z répondent aux définitions suivant la revendication 32 ; ou
(b) acylation d'un composé de formule IX, répondant à la définition précitée, avec un composé de formule 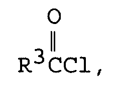 dans laquelle R³ répond à la définition suivant la revendication 1, puis réduction de l'amide résultant.

3. Procédé de préparation d'un composé de formule 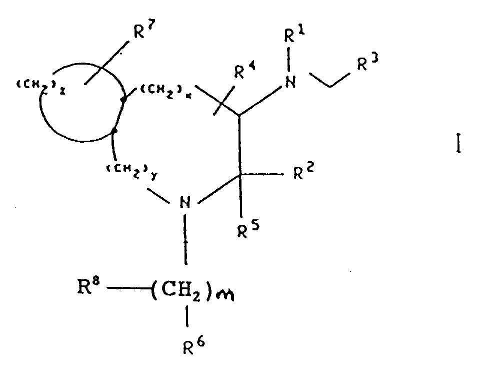 dans laquelle x est un nombre entier de zéro à quatre ;
y est un nombre entier de zéro à quatre ;
z est un nombre entier de un à six ;
le noyau contenant le groupe (CH₂)_{z} peut contenir zéro à trois doubles liaisons, et un des atomes de carbone du groupe (CH₂)_{z} peut être remplacé facultativement par l'oxygène, le soufre ou l'azote ;
m est un nombre entier de zéro à douze, et l'une quelconque des simples liaisons carbone-carbone du groupe (CH₂)ₘ peut être facultativement remplacée par une double ou triple liaison carbone-carbone et l'un quelconque des atomes de carbone dudit groupe (CH₂)ₘ peut être facultativement substitué avec R⁸ ;
R¹ représente l'hydrogène ou un groupe alkyle en C₁ à C₆ facultativement substitué avec un groupe hydroxy, alkoxy ou fluoro ;
R² représente un radical choisi entre
un radical cycloalkyle en C₁ à C₅ dans lequel un des atomes de carbone peut être facultativement remplacé par l'azote, l'oxygène ou le soufre ; un radical aryle choisi entre les radicaux phényle et naphtyle ; un radical hétéroaryle choisi entre les radicaux indanyle, thiényle, furyle, pyridyle, thiazolyle, isothiazolyle, oxazolyle, isoxazolyle, triazolyle, tétrazolyle et quinolyle ; les radicaux phényl(alkyle en C₂ à C₆), benzhydryle et benzyle, dans lesquels chacun desdits groupes aryle et hétéro-aryle et les groupements phényle desdits groupes benzyle, phényl-(alkyle en C₂ à C₆) et benzhydryle peuvent porter facultativement un ou plusieurs substituants choisis, indépendamment, entre des substituants halogéno, nitro, alkyle en C₁ à C₆, alkoxy en C₁ à C₆, trifluorométhyle,amino,alkylamino en C₁ à C₆, 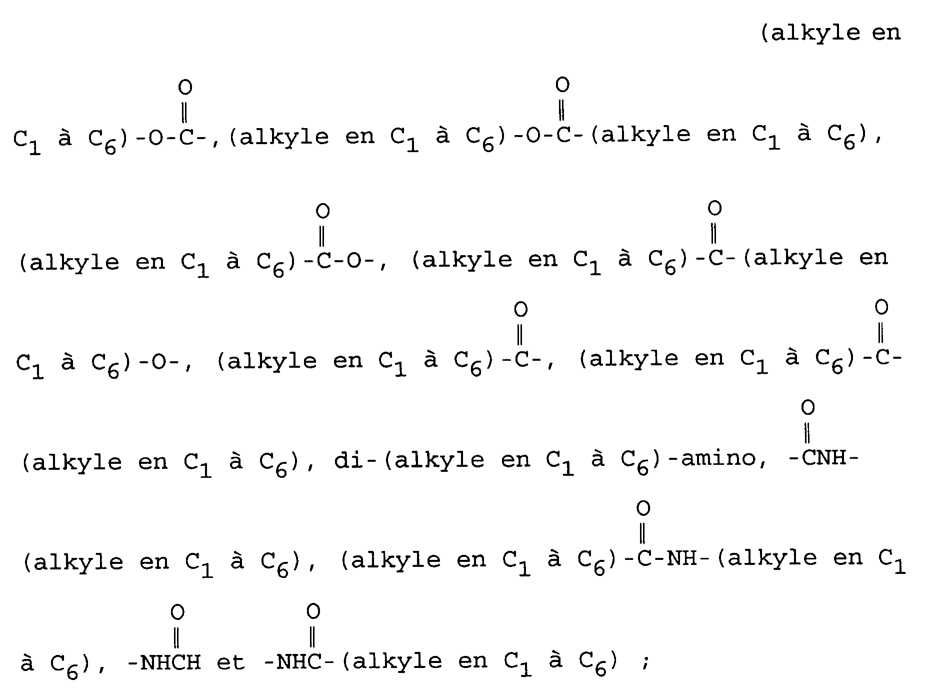 R⁵ représente l'hydrogène ou un groupe alkyle en C₁ à C₆ ;
ou bien R² et R⁵, conjointement avec l'atome de carbone auquel ils sont fixés, forment un noyau carbocyclique saturé ayant 3 à 7 atomes de carbone, dans lequel un desdits atomes de carbone peut être facultativement remplacé par l'oxygène, l'azote ou le soufre ;
R³ représente un radical aryle choisi entre les radicaux phényle et naphtyle ; un radical hétéro-aryle choisi entre les radicaux indanyle, thiényle, furyle, pyridyle, thiazolyle, isothiazolyle, oxazolyle, isoxazolyle, triazolyle, tétrazolyle et quinolyle ; ou un radical cycloalkyle ayant trois à sept atomes de carbone, dans lequel un desdits atomes de carbone peut être facultativement remplacé par l'azote, l'oxygène ou le soufre ; chacun desdits groupes aryle et hétéro-aryle pouvant porter facultativement un ou plusieurs substituants, et ledit radical cycloalkyle en C₃ à C₇ pouvant porter facultativement un ou deux substituants, lesdits substituants étant choisis, indépendamment, entre des substituants halogéno, nitro, alkyle en C₁ à C₆, alkoxy en C₁ à C₆, trifluorométhyle, phényle, amino, alkylamino en C₁ à C₆, 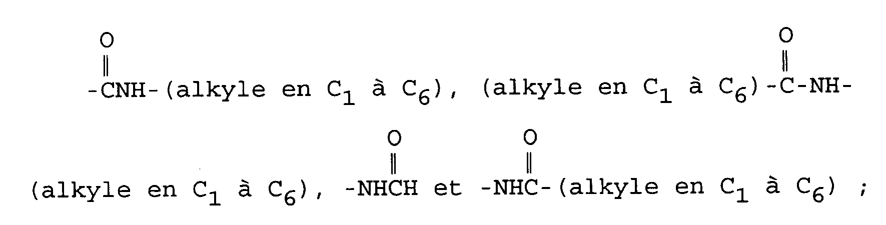 R⁴ peut être fixé à n'importe quel atome du noyau contenant de l'azote ayant un site de liaison disponible, et R⁷ peut être fixé à n'importe quel atome du noyau contenant le groupe (CH₂)_{z}, ayant un site de liaison disponible ;
R⁴, R⁶, R⁷ et R⁸ sont choisis chacun, indépendamment, entre l'hydrogène, des radicaux hydroxy, halogéno, amino, carboxy, carboxyalkyle, alkylamino en C₁ à C₆, di(alkyle en C₁ à C₆)-amino, alkoxy en C₁ à C₆, 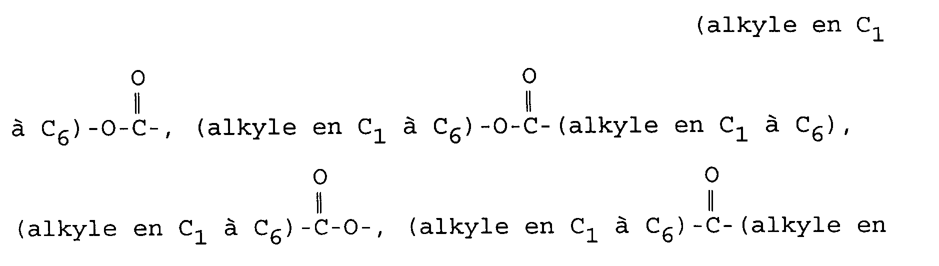 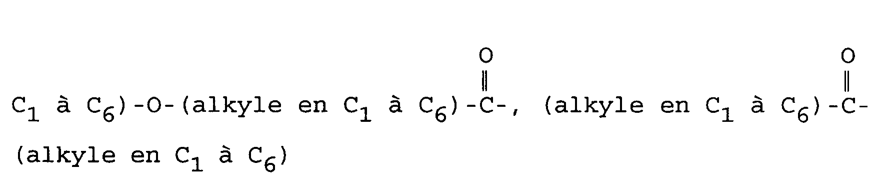 et les radicaux indiqués dans la définition de R² ;
ou un de ses sels pharmaceutiquement acceptables,
comprenant la réaction d'un composé de formule 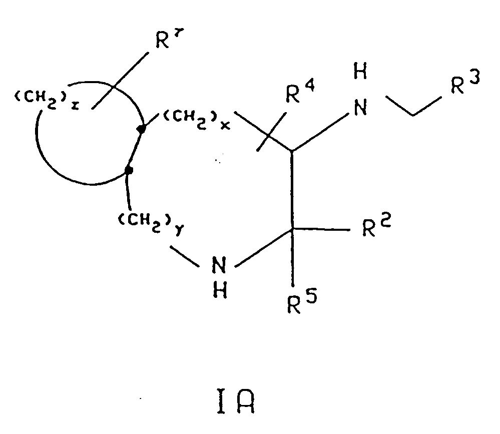 dans laquelle R², R³, R⁴, R⁵, R⁷, x, y et z répondent aux définitions précitées, avec un composé de formule R⁶-(CH₂)ₘ-X, dans laquelle m et R⁶ répondent aux définitions précitées et X représente un groupe partant, et dans laquelle une des simples liaisons carbone-carbone du groupe (CH₂)ₘ peut être facultativement remplacée par une double liaison carbone-carbone, et dans laquelle un des atomes de carbone du groupe (CH₂)ₘ peut être facultativement substitué avec R⁸, le groupe R⁸ répondant à la définition précitée.

4. Procédé de préparation d'un composé de formule 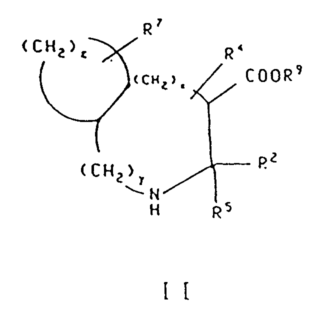 dans laquelle R⁹ représente un groupe alkyle en C₁ à C₄ et R², R⁴, R⁵, R⁷, x, y et z répondent aux définitions suivant la revendication 3, comprenant le chauffage d'un composé de formule 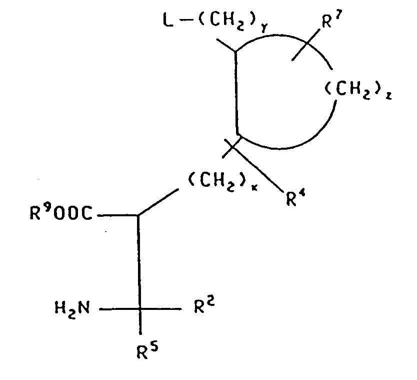 dans laquelle R⁹ représente un groupe alkyle en C₁ à C₄, L représente un groupe partant et R², R⁴, R⁵, R⁷, x, y et z répondent aux définitions suivant la revendication 3.

5. Procédé suivant la revendication 4, dans lequel le composé de formule V est obtenu par réaction d'un β-lactame de formule 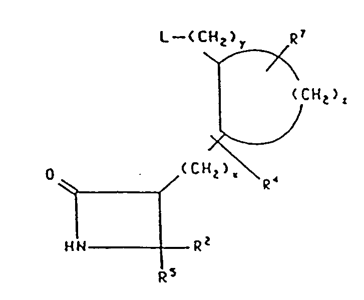 dans laquelle L représente un groupe partant et R², R⁴, R⁵, R⁷, x, y et z répondent aux définitions suivant la revendication 3, avec un acide.

6. Procédé suivant la revendication 1, qui produit un composé de formule IA dans laquelle y est égal à zéro, z est supérieur à un et R⁵ représente l'hydrogène.

7. Procédé suivant la revendication 2, qui produit un composé de formule IA, répondant à la définition suivant la revendication 1, dans laquelle y est égal à zéro, z est supérieur à un et R⁵ représente l'hydrogène.

8. Procédé de préparation d'un composé de formule 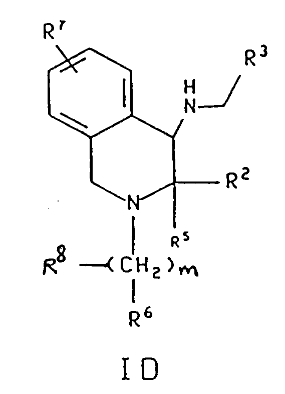 dans laquelle R², R³, R⁵, R⁶, R⁷, R⁸ et m répondent aux définitions suivant la revendication 3, comprenant la réduction d'un composé de formule 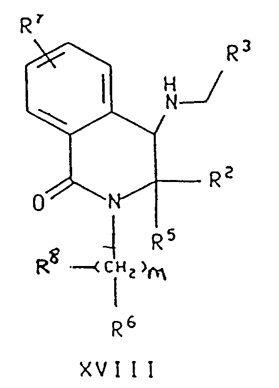 dans laquelle R², R³, R⁵, R⁶, R⁷, R⁸ et m répondent aux définitions suivant la revendication 3.

9. Procédé suivant la revendication 8, dans lequel le composé de formule XVIII est obtenu par :
(a) amination réductrice d'un composé de formule 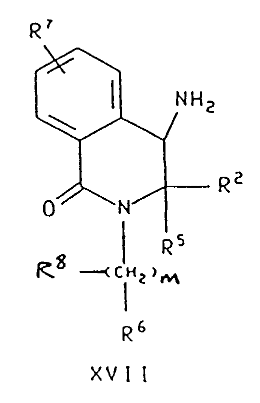 dans laquelle R², R⁵, R⁶, R⁷, R⁸ et m répondent aux définitions suivant la revendication 3 ; ou
(b) acylation d'un composé de formule XVII, répondant à la définition précitée, avec un composé de formule 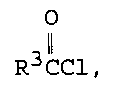 dans laquelle R³ répond à la définition suivant la revendication 3, puis réduction de l'amide résultant.
